# FASCICULE DE BREVET EUROPEEN

(11) **EP 3 186 359 B1**
(45) Date de publication et mention de la délivrance du brevet: **29.05.2019**
(21) Numéro de dépôt: 15760224.4
(22) Date de dépôt: 24.08.2015
(51) Int. Cl.: C12N 1/20, C12P 3/00, C12P 39/00

(54) **PROCÉDÉ DE PRODUCTION D'HYDROGÈNE PAR FERMENTATION OBSCURE À PARTIR DE BIOMASSES ISSUES DE LA FILIÈRE VITIVINICOLE, SANS APPORT DE CONSORTIUM MICROBIEN**
VERFAHREN ZUR HERSTELLUNG VON WASSERSTOFF MITTELS DUNKELFERMENTATION AUS BIOMASSE AUS DER WEINPRODUZIERENDEN INDUSTRIE OHNE VERWENDUNG EINES MIKROBIELLEN KONSORTIUMS
METHOD FOR PRODUCING HYDROGEN BY MEANS OF DARK FERMENTATION FROM BIOMASS FROM THE WINE-PRODUCING INDUSTRY, WITHOUT USING A MICROBIAL CONSORTIUM

(30) Priorité: 25.08.2014 EP 14306312; 15.10.2014 FR 1459895
(43) Date de publication de la demande: 05.07.2017
(73) Titulaire: Université de Strasbourg, 67000 Strasbourg (FR); Centre National de la Recherche Scientifique, 75794 Paris Cedex 16 (FR); Institut National Superieur Des Sciences Agronomiques de L'alimentation et de L'environment, 21079 Dijon Cedex (FR); Université de Bourgogne, 21078 Dijon Cedex (FR)
(72) Inventeur: ERNST, Barbara, F-67270 Gougenheim (FR); GOUGEON, Régis, F-21800 Quétigny (FR); FRANÇOIS-LOPEZ, Émilie, F-38400 SAINT-MARTIN-D'HÈRES (FR); DUMAS, Christine, F-67200 Strasbourg (FR); VUILLEUMIER, Stéphane, F-67000 Strasbourg (FR); ALEXANDRE, Hervé, F-21700 Meuilley (FR)
(74) Mandataire: Novagraaf Technologies
(86) Numéro de dépôt international: PCT/FR2015/052256
(87) Numéro de publication internationale: WO 2016/030623

(56) Documents cités:
- Francois-Lopez E. et al.: "VALORIZATION OF WINERY WASTE: BIOHYDROGEN PRODUCTION FROM ADAPTED FLORA BY DARK FERMENTATION", , 24 août 2014 (2014-08-24), XP055229204, Extrait de l'Internet: URL:http://ftp.servcbo.com/1408_WasteEng/a bstracts/Topic3/224-spc0224.pdf [extrait le 2015-11-13]
- MARONE ANTONELLA ET AL: "Vegetable waste as substrate and source of suitable microflora for bio-hydrogen production", RENEWABLE ENERGY, vol. 68, 14 February 2014 (2014-02-14), pages 6-13, XP028846823, ISSN: 0960-1481, DOI: 10.1016/J.RENENE.2014.01.013
- ANTONOPOULOU ET AL: "Biofuels generation from sweet sorghum: Fermentative hydrogen production and anaerobic digestion of the remaining biomass", BIORESOURCE TECHNOLOGY,, vol. 99, no. 1, 5 October 2007 (2007-10-05), pages 110-119, XP022285993, ISSN: 0960-8524, DOI: 10.1016/J.BIORTECH.2006.11.048
- FAVARO LORENZO ET AL: "Effects of inoculum and indigenous microflora on hydrogen production from the organic fraction of municipal solid waste", INTERNATIONAL JOURNAL OF HYDROGEN ENERGY, vol. 38, no. 27, 29 July 2013 (2013-07-29) , pages 11774-11779, XP028694453, ISSN: 0360-3199, DOI: 10.1016/J.IJHYDENE.2013.06.137
- JAE-HOON HWANG ET AL: "Feasibility of hydrogen production from ripened fruits by a combined two-stage (dark/dark) fermentation system", BIORESOURCE TECHNOLOGY,, vol. 102, no. 2, 18 August 2010 (2010-08-18), pages 1051-1058, XP028371519, ISSN: 0960-8524, DOI: 10.1016/J.BIORTECH.2010.08.047 [retrieved on 2010-08-24]

## Description

### Domaine technique

La présente invention se rapporte à la production d'hydrogène par fermentation obscure à partir de biomasses issues de la filière vitivinicole (bourbes, marcs, gâteaux de filtration, lies, marcs épuisés, eaux de rinçage des matériels de récolte, de tri et de vinification).

La présente invention trouve notamment une application dans le secteur de l'énergie avec la production d'un vecteur énergétique (hydrogène) à partir d'une biomasse agricole. Une autre application possible est la production d'hydrogène pour tout industriel utilisant l'hydrogène comme réactif sur son site et disposant de biomasses métabolisables.

Dans la description ci-dessous, les références entre crochets (**[ ]**) renvoient à la liste des références présentées à la fin du texte.

### Etat de la technique

La France est le plus gros producteur de vin : 50 millions d'hectolitres produits en moyenne par an entre 2000 et 2011 [1]. La génération de déchets organiques, y compris les sous-produits tels que les marcs de raisin, les bourbes, les lies, les rafles et boues déshydratées, est considérée comme l'un des problèmes les plus importants auxquels doit faire face l'industrie mondiale du vin [2]. Les marcs de raisin représentent 27% du rendement moyen du vignoble qui se situe autour de 7000 kg.ha⁻¹ en France, et sont composés de 4,5% de pépins de raisin, 19,5% de peau de raisin et 3% de rafles [3, 4]. Selon le règlement du Conseil européen 1234/2007, les marcs de raisin et lies de vin doivent être envoyés aux distilleries d'alcool sauf les petites industries vinicoles. Environ 850 000 tonnes de marcs de raisin sont envoyés chaque année aux distilleries françaises [5]. Compte tenu des quantités élevées produites chaque année par l'industrie du vin, du décalage croissant entre problèmes environnementaux et pénurie de combustibles fossiles, la valorisation des sous-produits et des déchets est inévitable. Les valorisations actuelles sont : les polyphénols, les tanins comme colles à bois, l'acide tartrique, les substrats végétaux, l'alimentation animale, le bioéthanol, etc...

A l'heure actuelle, 95% de l'hydrogène est produit à partir des combustibles fossiles par des procédés thermiques (ex. vaporéformage du gaz naturel). Parmi les possibilités de valorisation et dans le cadre de l'exploration de nouvelles sources d'énergie renouvelable, l'hydrogène (H₂) produit à partir des biomasses est un vecteur d'énergie propre et prometteur. En effet, l'hydrogène peut être utilisé en tant que vecteur énergétique dans des piles à combustible. Les technologies de production d'hydrogène à partir de la biomasse peuvent se décliner en deux catégories : (i) les processus thermochimiques : gazéification (procédé direct de production de H₂), pyrolyse, liquéfaction hydrothermale, et (ii) les processus biochimiques : photolyse directe/indirecte, fermentation [6], estérification, digestion. Ces derniers, bien que peu énergivores par rapport aux processus thermochimiques, produisent respectivement de l'éthanol, du biodiesel et du biogaz qui devront subir un traitement de reformage, étape thermique catalytique, pour produire de l'hydrogène. Parmi ces procédés, la photolyse de l'eau utilisant des algues ou des cyanobactéries et la fermentation obscure (*dark fermentation*) de composés organiques avec des micro-organismes, agissant comme des biocatalyseurs, permettent de produire de l'hydrogène à basse température (37°C) en évitant les étapes de reformage catalytique ou de gazéification qui sont très énergivores. Le rendement énergétique maximum (conversion de la biomasse en hydrogène) du procédé de fermentation obscure est de 67% (en pratique de 15 à 33%), valeur bien supérieure à celle du procédé de photolyse de l'eau.

La production de H₂ par des procédés biologiques, tels que la fermentation obscure, est moins énergivore et peut être réalisée dans des conditions non stériles, en utilisant des cultures mixtes et des déchets organiques peu coûteux comme substrats [7]. La conversion de déchets organiques en H₂ implique une communauté microbienne complexe utilisant différentes voies métaboliques. Le rendement théorique maximal en H₂ à partir de 1 mol d'hexose est de 4 mol_{H2}.mol⁻¹ₕₑₓₒₛₑ via la voie acétate (équation 1) et 2 mol_{H2.}mol⁻¹ ₕₑₓₒₛₑ via la voie butyrate (équation 2) [8,9]. Le rendement expérimental peut atteindre une limite de 2,5 mol_{H2.}mol⁻¹ ₕₑₓₒₛₑ (équation 3) lorsque différentes voies métaboliques sont impliquées (combinaison de la voie acétate et butyrate) [10]. Le rapport molaire butyrate/acétate (B/A) peut estimer la conformité de l'équation 3 où l'optimum est 3/2. Ces rendements sont possibles seulement si la pression partielle en H₂ est basse et si les micro-organismes consommant H₂ sont inhibés, tels que les archées méthanogènes ou les bactéries homoacétogènes (équation 4). Le traitement thermique est l'un des traitements les plus utilisés pour inhiber les micro-organismes consommant H₂ et augmente spécifiquement la croissance de bactéries sporulées produisant H₂. En dehors des voies de production et de consommation de H₂, les voies compétitives telles que la voie éthanolique (équation 5) peuvent être impliquées au cours de la fermentation anaérobie.

C₆H₁₂O₆ + 2H₂0 → 2CH₃COOH + 2CO₂ + 4H₂ (ΔG⁰₂₉₈ = -215kJ.mol⁻¹) (1)

C₆H₁₂O₆ → CH₃CH₂CH₂COOH + 2CO₂ + 2H₂ (ΔG⁰₂₉₈ = -264kJ.mol⁻¹) (2)

4C₆H₁₂O₆ + 2H₂0 → 3CH₃CH₂CH₂COOH + 2CH₃COOH + 8CO₂ + 10H₂ (ΔG⁰₂₉₈ =-252kJ.mol⁻¹) (3)

2CO₂ + 4H₂ → CH₃COOH + 2H₂O (ΔG⁰₂₉₈ = -94kJ.mol⁻¹) (4)

C₆H₁₂O₆ → 2CH₃CH₂OH + 2CO₂ (ΔG⁰₂₉₈ = -235kJ.mol⁻¹) (5)

Cependant, dans la littérature, les boues ou eaux usées sont souvent utilisées comme inoculum, et très peu d'auteurs ont étudié la fermentation obscure à partir de déchets organiques sans l'utilisation de cultures mixtes additionnelles.

Marone et al. (Renewable Energy 68:6-13, 2014) décrit la production d'hydrogène par fermentation obscure de déchets de légumes, sans traitement thermique, et sans l'ajout d'un inoculum.

Par ailleurs, le coût de l'hydrogène est principalement lié à son transport. En effet, le coût de production industrielle de l'hydrogène s'élève à 2 € par kg d'hydrogène obtenu par vaporéformage du méthane ; à ce coût s'ajoute toutefois celui de la distribution jusqu'à l'utilisateur, lui-même fonction des distances à parcourir et de la densité des lieux de livraison. C'est dans une optique de décentralisation de la production de l'hydrogène que le procédé de fermentation obscure de biomasses ou d'autres procédés de production d'hydrogène pourrait devenir compétitif ; la production décentralisée d'hydrogène à partir du gaz naturel aurait dans cette configuration un coût minimum de 6 € par kg d'hydrogène [11].

Il existe donc un réel besoin de mettre au point un nouveau procédé de production d'hydrogène écologique, efficace et peu énergivore palliant les défauts, inconvénients et obstacles de l'art antérieur, en particulier d'un procédé permettant de produire de l'hydrogène avec un bon rendement, sans production de méthane, sans avoir à faire l'usage d'inoculum de cultures mixtes, et de réduire les coûts.

### Description de l'invention

Les Inventeurs ont ainsi mis en évidence de manière tout à fait inattendue une production efficace d'hydrogène sur diverses biomasses vitivinicoles issues de plusieurs terroirs sans apport d'inoculum microbien, sans apport nutritionnel additionnel ni prétraitement thermique ; alors que les travaux décrits dans la littérature utilisent systématiquement un inoculum de cultures mixtes avec des rendements inférieurs à ceux obtenus avec le procédé de l'invention.

Le procédé de l'invention est constitué des premiers travaux de valorisation de biomasses vitivinicoles, non encore explorées pour certaines à ce jour (par exemple, bourbes, marcs, eaux de rinçage des pressoirs, eaux de rinçage des cuves de débourbage), pour la production d'hydrogène par le processus biologique de fermentation obscure. De façon globale, le procédé de fermentation obscure est peu énergivore comparativement aux procédés de valorisation actuels comme la distillation des marcs et des bourbes pour la production d'éthanol. La consommation énergétique pour la mise en oeuvre de la fermentation repose essentiellement sur le maintien du réacteur en isotherme (température à 37°C) et la mise en suspension de la biomasse par agitation.

Le procédé de l'invention permet ainsi de produire de l'hydrogène avec de bons rendements sans production de méthane, sans avoir à faire usage d'inoculum de cultures mixtes mais en utilisant le consortium microbien endogène présent dans la biomasse vitivinicole, et sans prétraitement thermique. En effet, le procédé de production met en évidence une sélection de micro-organismes producteurs d'hydrogène forcée par les conditions de mise en oeuvre du bioréacteur semi-batch (extraction des gaz produits, fonctionnement en anaérobiose et à l'obscurité, conditions de pH et température contrôlées). Les résultats montrent que les consortia microbiens présents dans la biomasse sont les plus adaptés pour métaboliser les sucres constitutifs de celle-ci en hydrogène (bourbes : rendement moyen de 2,0 mol H₂/mol hexose ; marc de raisin : rendement moyen de 1,4 mol H₂/mol hexose). En effet, les données de la littérature sur la fermentation obscure de raisins mûrs en présence de cultures mixtes (consortium microbien exogène) rapportent un rendement de seulement 0,1 mol H₂/mol hexose [12], très largement inférieur à ceux obtenus par le procédé de l'invention. Par ailleurs, hormis la régulation du pH du milieu réactionnel à une valeur optimisée, aucun traitement thermique n'est nécessaire pour sélectionner les micro-organismes recherchés dans le procédé de l'invention. Alors que d'une façon générale, dans les procédés de fermentation de l'art en présence d'un consortium microbien complexe (tel que l'on peut le rencontrer dans les boues de station d'épuration (STEP)), un traitement thermique avant fermentation est nécessaire afin d'inhiber l'activité des organismes hydrogénotrophes ou compétiteurs de la production d'hydrogène et ainsi augmenter le rendement de production en hydrogène.

La présente invention a donc pour objet un procédé de production d'hydrogène comprenant la fermentation obscure de biomasses vitivinicoles, sans ajout d'inoculum microbien et sans prétraitement thermique.

On entend par « fermentation obscure » au sens de la présente invention, la fermentation anaérobie qui consiste en la transformation par fermentation de substrats organiques en biohydrogène. Il s'agit d'un processus complexe réalisé par divers groupes de micro-organismes, impliquant une série de réactions biochimiques utilisant trois étapes similaires de la digestion anaérobie. La fermentation obscure diffère de la photofermentation en ce qu'elle se déroule en l'absence de lumière.

Selon un mode de réalisation particulier du procédé de l'invention, aucun apport nutritionnel additionnel n'est apporté à la biomasse.

Selon un mode de réalisation particulier du procédé de l'invention, la biomasse vitivinicole est choisie parmi les bourbes de raisin, les marcs de raisin, les lies de vin, les marcs épuisés, les gâteaux de filtration, les eaux de rinçage des matériels de récolte, de tri et de vinification. La biomasse vitivinicole peut provenir d'une ou plusieurs sources différentes, par exemple de bourbes de raisin et d'eaux de rinçage. Cette biomasse, issue de la filière viticole, concerne tous les raisins utilisés pour la vinification par pressurage de la vendange quelle que soit la couleur du raisin sans restriction de cépage ou de terroir. Par exemple, la biomasse vitivinicole peut être issue de bourbes, de marcs de raisin, et/ou des eaux de rinçage des pressoirs, de gâteaux de filtration, de lies de vin, provenant de diverses terroirs (Alsace, Bourgogne, et/ou eaux de rinçage des pressoirs ;et dont le raisin provient de cépages diversifiés (Muscat, Auxerrois, Pinot gris, Gewurztraminer, Riesling, Pinot noir, Chardonnay, Pinot noir/Chardonnay ou Aligoté).

Selon un mode de réalisation particulier du procédé de l'invention, la fermentation est réalisée à 37°C ± 2°C.

Selon un mode de réalisation particulier du procédé de l'invention, la biomasse vitivinicole peut être conservée par réfrigération ou par congélation avant utilisation.

Selon un mode de réalisation particulier du procédé de l'invention, le pH de la biomasse est maintenu supérieur à 5,5 ± 0,2 pendant la fermentation.

Selon un mode de réalisation particulier du procédé de l'invention, le gaz produit par la fermentation de la biomasse est extrait par un gaz de balayage (azote par exemple).

Selon un mode de réalisation particulier du procédé de l'invention, ledit procédé peut comprendre en outre une étape d'isolement du consortium microbien contenu dans la biomasse fermentée ou en fermentation.

La présente invention a également pour objet un procédé d'obtention du consortium microbien, issu de la biomasse vitivinicole, susceptible d'être obtenu par le procédé de l'invention. Par exemple, le consortium microbien comprend majoritairement une flore microbienne appartenant aux genres *Enterobacter*/*Klebsiella*/*Kluyvera*/*Buttiauxella* pour les marcs et aux espèces *Clostridium saccharobutylicum, Clostridium spp. (saccharoperbutylacetonicum, beijerinckii, puniceum, diolis, roseum*), *Streptococcus spp.* (*lutetiensis, infantarius, equinus, luteciae*), *Clostridium butyricum* pour les bourbes.

D'autres avantages pourront encore apparaître à l'homme du métier à la lecture des exemples ci-dessous.

### Brève description des figures

- La figure 1 représente la classification taxonomique aux niveaux du phylum et de la famille (a et b) des échantillons prélevés au cours de la fermentation lors du test sur la biomasse bourbes Pinot Gris d'Alsace (vendanges 2014) stockée à -20°C pendant 47 jours (c).
- La figure 2 représente les performances de production d'hydrogène obtenues en fonction de la durée de stockage à -20°C de la biomasse bourbes d'Alsace Pinot Gris (vendanges 2014) : 35, 47, 63 et 115 jours.
- La figure 3 représente la classification taxonomique au niveau de la famille réalisée à partir des échantillons prélevés à 24 h de fermentation lors des tests '35 jours', '47 jours' et '63 jours' pour la biomasse bourbes d'Alsace (Pinot Gris) (vendanges 2014).La figure 4 représente les performances de production d'hydrogène obtenues en fonction de la charge de biomasse.
- La figure 5 représente la classification taxonomique au niveau de la famille réalisée à partir des échantillons prélevés à 24 h de fermentation lors des tests '14 g DCO/L', '28 g DCO/L', '60 g DCO/L' et '92 g DCO/L'.
- La figure 6 représente les performances de production d'hydrogène obtenues en fonction du prétraitement et de la nature du gaz de balayage.
- La figure 7 représente la classification taxonomique au niveau de la famille réalisée à partir des échantillons prélevés à 24 h de fermentation lors des tests 'sans pt', 'ptf' et 'ptf + ptc'.

### EXEMPLES

### EXEMPLE 1 : MATERIELS ET METHODES

La fermentation obscure a été réalisée dans un réacteur de 1L à double enveloppe placé à l'obscurité et doté d'un système d'extraction des gaz, qui permet à la fois d'assurer les conditions d'anaérobiose et de réduire au minimum la pression partielle en hydrogène, celui-ci étant inhibiteur de l'activité microbienne.

### Dispositif expérimental - Equipements

Les tests ont été réalisés dans un réacteur de 1L (Büchi AG) agité, fonctionnant à pression atmosphérique. Il est équipé d'une double enveloppe connectée à un bain thermostaté (Bioblock Scientific) permettant de contrôler la température dans le bioréacteur. Le pH du milieu réactionnel est suivi *in situ* à l'aide d'une sonde pH-métrique et est ajusté avec un régulateur de pH (Hanna Instruments BL 7916) connecté au réacteur, par injection d'une solution de soude. Le réacteur est alimenté par un gaz de balayage (azote) dont le débit est régulé par un débitmètre massique (Brooks 5850^{E}) connecté à un régulateur (Brooks Instruments 0254). Les gaz produits au cours de la fermentation, entrainés par le flux d'azote en sortie du bioréacteur, circulent dans deux pièges placés en série, réfrigérés à 0°C à l'aide d'un cryostat (Huber TC40) afin de piéger par condensation les composés volatils provenant du milieu réactionnel liquide. La production de gaz est analysée en ligne par un micro-chromatographe en phase gazeuse (Agilent M200). Le réacteur est équipé d'une vanne de purge pour réaliser les prélèvements nécessaires à la quantification des métabolites identifiés et analysés à l'aide d'un chromatographe en phase gazeuse avec un détecteur à ionisation de flamme (Agilent GC 7890Q).

### Protocole de mise en oeuvre et conditions expérimentales

### Conditionnement et préparation de la biomasse

Les échantillons (marcs, bourbes, eaux de rinçage) ont été collectés sur différents terroirs, puis stockés à -20°C pour limiter le démarrage des processus fermentaires, sauf pour le lot d'échantillons «frais » stockés à 4°C.

Pour les eaux de rinçage, un volume de 700mL d'eaux de rinçage a été placé dans le bioréacteur.

Pour les bourbes, un volume de 650mL d'eau du réseau de la Communauté Urbaine de Strasbourg (CUS) ou d'eaux de rinçage a été ajouté à 50mL de bourbes préalablement placées dans le bioréacteur pour obtenir un volume final de 700mL. Pour les essais préliminaires, des boues activées (zone anoxique) de la station d'épuration (boues de STEP) de la Communauté Urbaine de Strasbourg ont substituées l'eau du réseau de la CUS afin d'ajouter un inoculum externe.

Pour les marcs, un volume de 650mL d'eau du réseau de la CUS ou d'eaux de rinçage a été ajouté à 50g de marcs de raisin préalablement placés dans le bioréacteur pour obtenir un volume final de 700mL.

### Test de fermentation

Le pH du milieu réactionnel (bourbes diluées, marcs dilués ou eaux de rinçage) a été initialement ajusté à 7,0 dans le bioréacteur à l'aide d'une solution d'hydroxyde de sodium à 3M. Lorsqu'un prétraitement thermique (ht) a été appliqué (essais préliminaires et marcs dilués), le milieu réactionnel a subi une montée en température de 15min jusqu'à 70°C sous atmosphère d'air statique et un palier d'une heure à cette température, au bout duquel la température a décru jusqu'à 37°C. Le milieu réactionnel a alors été placé sous flux d'azote avec un débit de 50mL_{N2}/min, à une température contrôlée à 37°C et sous agitation à 220tr/min puis à l'obscurité. Ce flux d'azote a permis à la fois d'assurer des conditions anaérobies et d'extraire les gaz produits. Ainsi, la pression partielle en hydrogène n'a pas excédé 0,05bar. Lorsque la régulation de pH a été appliquée, 0,5mL de solution de soude à 1M (sauf pour les essais à 3M) a été injecté quand la valeur de pH a été inférieure à 5,50±0,2.

Les tests de fermentation ont été réalisés sans aucun apport de milieu nutritif additionnel (hormis la biomasse vitivinicole).

### Analyses

### Analyses des gaz

L'analyse en ligne des gaz a été assurée par un microchromatographe Agilent M200 composé de deux modules équipés de détecteurs à conductivité thermique (TCD). Le premier module a permis la détection de N₂, O₂, CH₄ et H₂ et est constitué d'une colonne de type tamis moléculaire 5Å (10m x 0,32m x 30µm) utilisant l'argon comme gaz vecteur. Le deuxième module a permis de détecter et quantifier CO₂ et CH₄ grâce à une colonne capillaire PoraPlot U (8m x 0,32m x 10µm) constituée de divinylbenzène éthylène glycol comme phase stationnaire et utilisant l'hélium comme gaz vecteur.

### Analyses des métabolites

La composition en acides gras volatils (AGV) et en alcools de la phase liquide : acétate (C2), propionate (C3), butyrate et iso-butyrate (C4 et iC4), valérate et iso-valérate (C5 et iC5), éthanol et butanol, a été analysée par chromatographie en phase gazeuse (Agilent 7890Q GC) avec un détecteur à ionisation de flamme (FID) et une colonne FFAP (15m x 0,1m x 0,1 µm) utilisant le polyéthylène glycol modifié avec de l'acide nitrotérephtalique comme phase stationnaire.

Les échantillons ont été préalablement centrifugés à 15000tr/min pendant 15min, puis les surnageants ont été filtrés sur des filtres seringues (diamètre de pores de 0,22µm). Chaque échantillon, dilué au ¼, a ensuite été mélangé à de l'acide trifluoroacétique à une concentration de 0,03M en ratio volumique 1:1 avant toute injection dans le GC-FID pour être à un pH inférieur à 2. L'hélium a été utilisé comme gaz vecteur à un débit de 0,4mL/mn et une pression de 3,6bar.

### Analyses des sucres

L'analyse des sucres totaux a été réalisée par dosage colorimétrique à l'anthrone. La quantité de sucres consommés a été calculée en effectuant la différence entre la quantité initiale et la quantité finale mesurées dans les surnageants obtenus par centrifugation à 4500 tr/min pendant 30 min des prélèvements issus du bioréacteur. Pour la mesure de la quantité initiale en sucres contenues dans les marcs de raisin, un traitement de 4 heures aux ultrasons a été réalisé.

### Analyse qualitative de la microflore par séquençage haut débit (réalisée en externe par INRA Transfert Environnement, Narbonne)

L'étude des populations microbiennes présentes dans les matrices ADN a été effectuée par pyroséquençage Roche 454 (séquenceur : Roche GS FLX + System XLR 70 sequencing chemistry) et par séquençage Illumina MiSeq du gène de l'ARN ribosomique 16S présent dans tous les génomes microbiens. Les analyses bioinformatiques ont permis de traiter les fichiers de séquences brutes et de réaliser l'identification de chaque séquence sur la base de la taxonomie *SILVA.* Un regroupement a été effectué sur la base de 100% d'homologie entre deux séquences et la séquence la plus proche dans la base de données *Genbank* a été identifiée.

### EXEMPLE 2 : POTENTIALITE DE LA BIOMASSE « BOURBES » EN TANT QUE SUBSTRAT ET MISE EN EVIDENCE DE LA PRESENCE D'UN CONSORTIUM MICROBIEN ENDOGENE PRODUCTEUR D'HYDROGENE

La potentialité de la biomasse « bourbes » a été étudiée pour la production d'hydrogène en tant que substrat avec pour inoculum les cultures mixtes (CM) urbaines issues des boues activées (zone anoxie) de la station d'épuration (boues STEP) de la Communauté Urbaine de Strasbourg. L'effet du prétraitement thermique (ht) de la biomasse « bourbes » avec cultures mixtes et sans cultures mixtes sur la production d'hydrogène a été testé.

La première approche liée à notre expérience de l'utilisation des boues de STEP a été de diluer la biomasse « bourbes » dans les boues de station d'épuration (MES = 4 g/L) et d'appliquer un prétraitement thermique à 70°C permettant l'inhibition de bactéries hydrogénotrophes, consommatrices d'hydrogène ou d'archées méthanogènes, productrices de méthane ; en effet, sans traitement thermique les cultures mixtes avec ajout d'un substrat modèle sont très peu productives d'hydrogène (rendement en H₂ de 0,23 mol H₂/mol d'hexoses consommés).

Les résultats obtenus, présentés dans le tableau 1, sur des bourbes provenant d'Alsace (cépage Pinot gris) (PG+CM^{ht}) (test 1) et de Bourgogne (B+CM^{ht}) (test 5) ont montré non seulement un volume produit d'hydrogène élevé lié à une concentration en matières métabolisables (sucres) importante mais surtout un rendement plus élevé respectivement de 2,31 et 2,21 mol H₂.mol⁻¹ d'hexoses consommés, que celui obtenu avec un substrat modèle (2,10 mol H₂.mol⁻¹ hexoses).

La seconde approche a alors été de tester la biomasse « bourbes » diluée dans les boues de STEP sans appliquer de traitement thermique (échantillons PG+CM et B+CM) (tests 2, 6). Il a été observé un rendement en hydrogène certes plus faible que pour la biomasse traitée thermiquement mais tout de même très significatif, de 2,1 à 3,6 fois plus élevé que celui obtenu pour les cultures mixtes additionnées d'un substrat modèle et non traitées thermiquement ; la production en gaz étant en faveur du dioxyde de carbone (rapport molaire H₂/CO₂ faible).

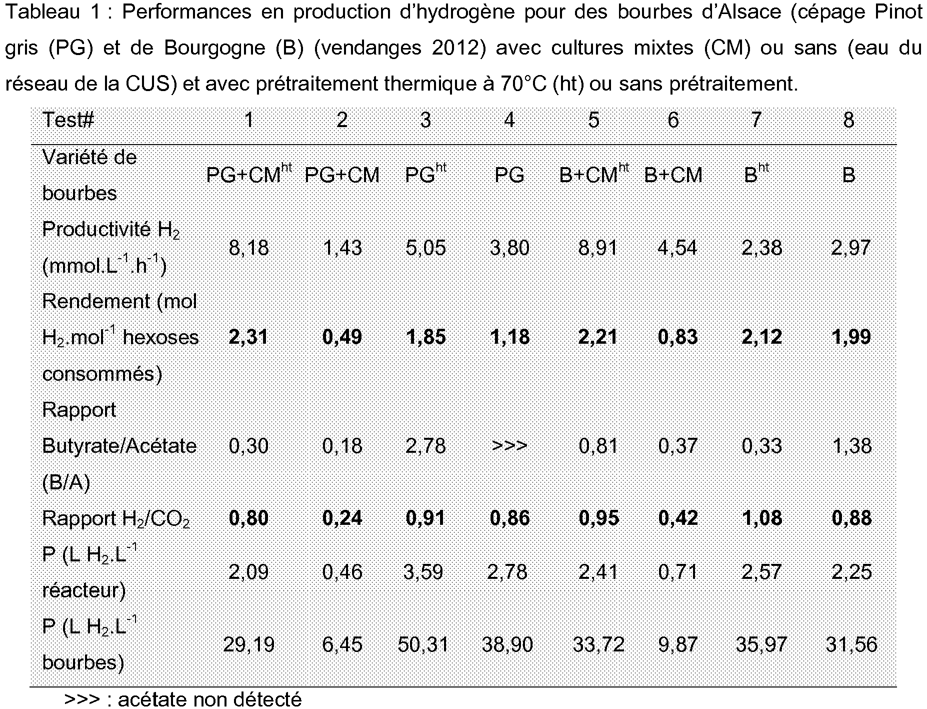

Cette production d'hydrogène révélatrice d'une activité microbienne autre que celle des boues de STEP et productrice d'hydrogène a orienté les expériences vers une troisième approche par la mise en oeuvre de la biomasse « bourbes » dans le bioréacteur en l'absence de cultures mixtes et diluées dans l'eau du réseau de la Communauté Urbaine de Strasbourg.

Les résultats de la biomasse « bourbes » diluées avec prétraitement thermique (PG^{ht} et B^{ht}) (tests 3, 7) sans aucun apport d'inoculum microbien ont montré une activité remarquable avec un rendement en hydrogène inférieur mais proche de celui obtenu pour la biomasse « bourbes » avec cultures mixtes, avec de plus, un volume d'hydrogène produit par litre de réacteur très supérieur spécifiquement pour la biomasse d'Alsace (+72%). Ces résultats ont mis en évidence une activité fermentaire endogène dans ce type de biomasse, non explorée dans la littérature. Le rapport molaire H₂/CO₂ plus élevé (+ 13,7% pour les deux types de bourbes) a révélé la présence d'un consortium bactérien sélectif à la production d'hydrogène.

Dans une optique de minimiser les dépenses énergétiques liées au prétraitement thermique lors du procédé, des tests ont été réalisés avec la biomasse « bourbes » diluée dans l'eau sans traitement thermique (échantillons PG et B) (tests 4, 8).

Les résultats ont mis en évidence un rendement en hydrogène nettement plus élevé, de 58,5% et 58,3% respectivement pour la biomasse d'Alsace et de Bourgogne que ceux des bourbes additionnées des boues de la STEP, avec une production en hydrogène par litre de réacteur plus importante (test 4 par rapport à test 2) ou équivalente (test 8 par rapport à test 6). Ces premiers essais probants ont attesté de l'existence de consortia microbiens dans la biomasse vitivinicole de type « bourbes » ; celle-ci jouant le rôle à la fois de substrat et d'inoculum microbien lors de la réaction de fermentation, non nécessairement mésophile et sans sélection microbienne spécifique. Cette forte potentialité, démontrée pour la première fois, a donné lieu aux essais suivants pour lequel aucun traitement thermique à 70°C n'a été nécessaire.

### EXEMPLE 3 : PERFORMANCE EN PRODUCTION D'HYDROGENE POUR LA BIOMASSE « MARCS »

Les marcs de raisin issus des vendanges 2012 provenant de différents cépages issus de différents terroirs (Alsace (Domaine Pfister, Dahlenheim) et Bourgogne (Domaine de l'Université de Bourgogne)) ont été utilisés en tant que substrat et inoculum microbien. Les paramètres testés sont l'impact des composés ligno-cellulosiques provenant des rafles des grappes de raisin sur la production d'hydrogène ainsi que le prétraitement thermique à une température de 70°C. Les résultats de production sont consignés dans le tableau 2.

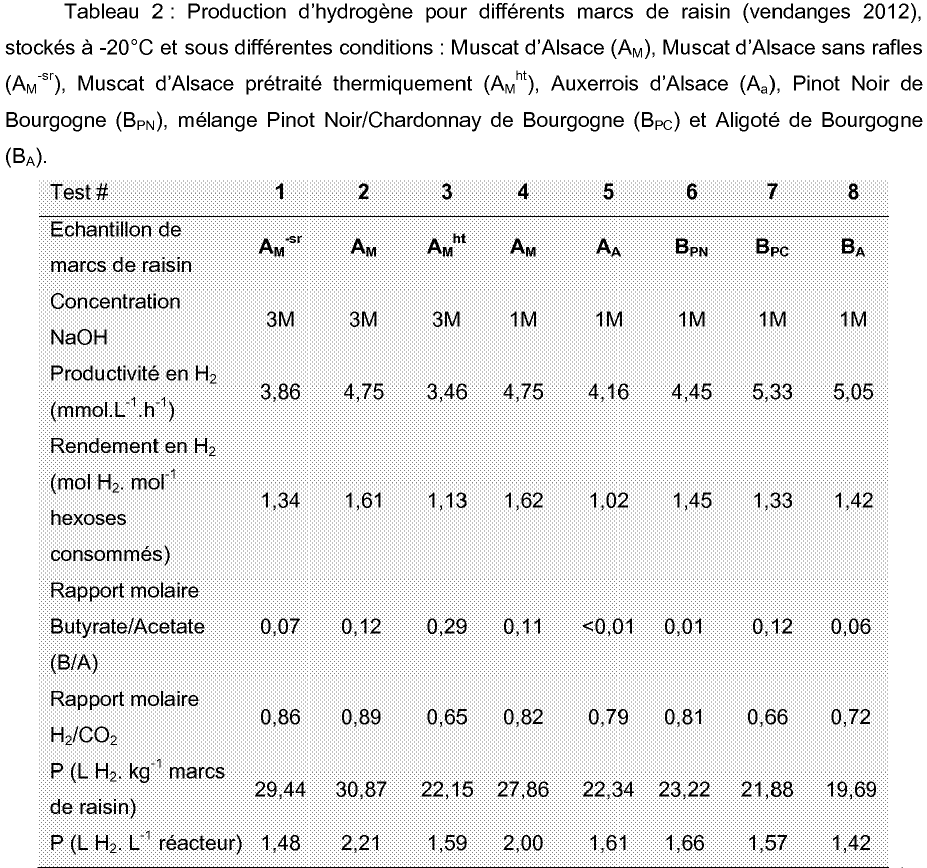

La comparaison de la biomasse composée de marcs de Muscat (A_{M}^{ht}) prétraitées thermiquement ou non (A_{M}) (tests 3 et 2) a mis en évidence une productivité et un rendement plus faible en hydrogène pour la biomasse prétraitée avec un rapport molaire butyrate/acétate significativement plus élevé pouvant expliquer un rapport H₂/CO₂ nettement plus faible (0,65 contre 0,89). Ce résultat a conforté l'idée selon laquelle l'étape de traitement à 70°C opérant potentiellement une sélection de bactéries thermophiles n'apportait pas d'effet positif pour ce type d'échantillon et constitue une étape énergivore supplémentaire.

Il est à remarquer que la concentration de la solution en soude injectée (3M et 1M) pour la régulation de pH (tests 2, 4) a eu peu d'effet sur les performances de production en hydrogène et a apporté ici l'information d'une très bonne reproductibilité quant à la vitesse de production en hydrogène (productibilité en hydrogène à 4,75 mmol.L⁻¹.h⁻¹).

Les différentes variétés de marcs de raisin en fonction de leur cépage (tests 4, 5, 6, 7, 8) ont présenté une productivité en hydrogène du même ordre de grandeur avec une valeur moyenne de 4,57 ± 0,51 mmol.L⁻¹.h⁻¹ ; un rendement en hydrogène moyen de 1,37 ± 0,22 mol H₂.mol⁻¹ d'hexoses consommés, un rapport molaire H₂/CO₂ de 0,76 ± 0,07 et une production d'H₂ de 23,00 ± 3,01 L H₂.kg⁻¹ de marcs de raisins. Les performances les plus élevées ont été obtenues pour les marcs de Muscat (A_{M}).

Il est à noter que ces résultats obtenus à partir de microflores indigènes non traités thermiquement sont comparables (voire supérieurs) aux résultats obtenus avec des marcs de pommes avec addition de cultures mixtes et prétraitement thermique (18,48 L H₂.kg⁻¹ de marcs de pommes) [13].

Les marcs de raisin issus des vendanges 2013 provenant de différents cépages issus de différents terroirs : Alsace (Domaine Pfister, Dahlenheim) et Bourgogne (Domaine des Poncétys du Lycée Viticole de Macon-Davayé) ont été utilisés en tant que substrat et inoculum microbien. Les paramètres testés pour les échantillons de marcs d'Alsace sont la reproductibilité d'une année sur l'autre pour un même cépage (Muscat), la variation de production en fonction de la nature du cépage sur un même domaine et pour ceux de Bourgogne, l'impact du mode de traitement de la vigne pour un même cépage (Chardonnay) et un même domaine. Les résultats de production sont consignés dans le tableau 3.

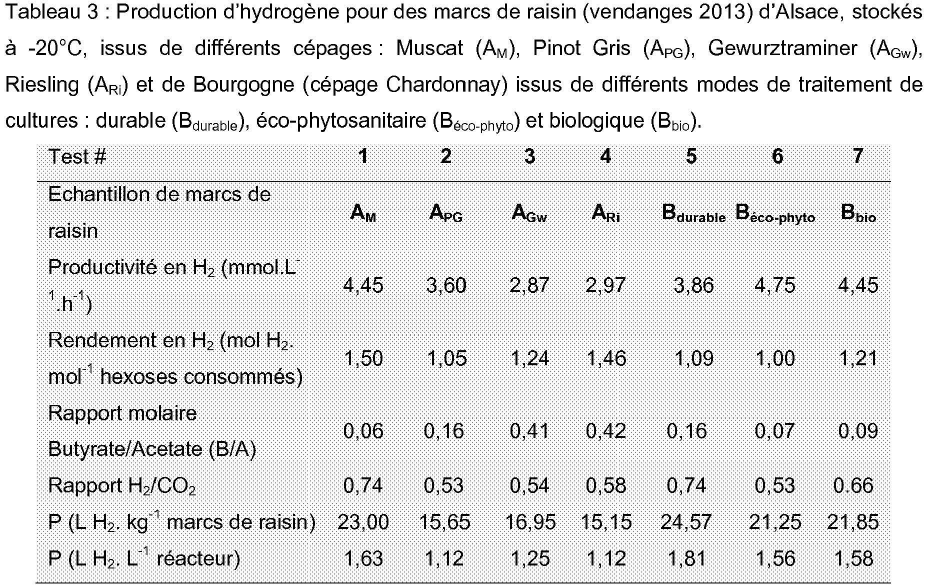

On remarque que, pour des marcs de raisin d'un même cépage (Muscat) sur un même domaine vendangés en 2012 (test 4 tableau 2) et 2013 (test 1 tableau 3), les paramètres de production et de productivité en hydrogène sont très proches (écart de 6,2% sur le rendement en H₂, 6,3 % sur la productivité et 16,8% sur le rapport H₂/CO₂). Ce résultat a mis en évidence la régularité de la production d'une année sur l'autre tout au moins sur cet essai et la très bonne sensibilité avec laquelle ont été effectuées les mesures.

Les différentes variétés de marcs de raisin d'Alsace en fonction de leur cépage (tests 1, 2, 3, 4) ont présenté une productivité en hydrogène avec une valeur moyenne de 3,47 ± 0,73 mmol.L⁻¹.h⁻¹, un rendement en hydrogène moyen de 1,32 ± 0,21 mol H₂.mol⁻¹ d'hexoses consommés, un rapport molaire H₂/CO₂ de 0,60 ± 0,10 et une production d'H₂ de 17,69 ± 3,62 L H₂.kg⁻¹ de marcs de raisin. Les meilleurs résultats ont été obtenus pour le cépage Muscat.

Les différents modes de traitement des cultures appliqués aux marcs de Chardonnay de Bourgogne (tests 5, 6, 7) ont eu peu impact sur le rendement en hydrogène. Il est à noter pour les 3 échantillons une production par kg de marcs de raisin également proche.

Les paramètres supplémentaires testés sur les marcs d'Alsace sont l'effet de l'addition des eaux de rinçage des pressoirs aux marcs « frais » stockés à froid (4°C) sur des durées (en jours) équivalentes pour différents cépages et la comparaison avec les échantillons conservés à -20°C (tf). Les résultats de production sont consignés dans le tableau 4.

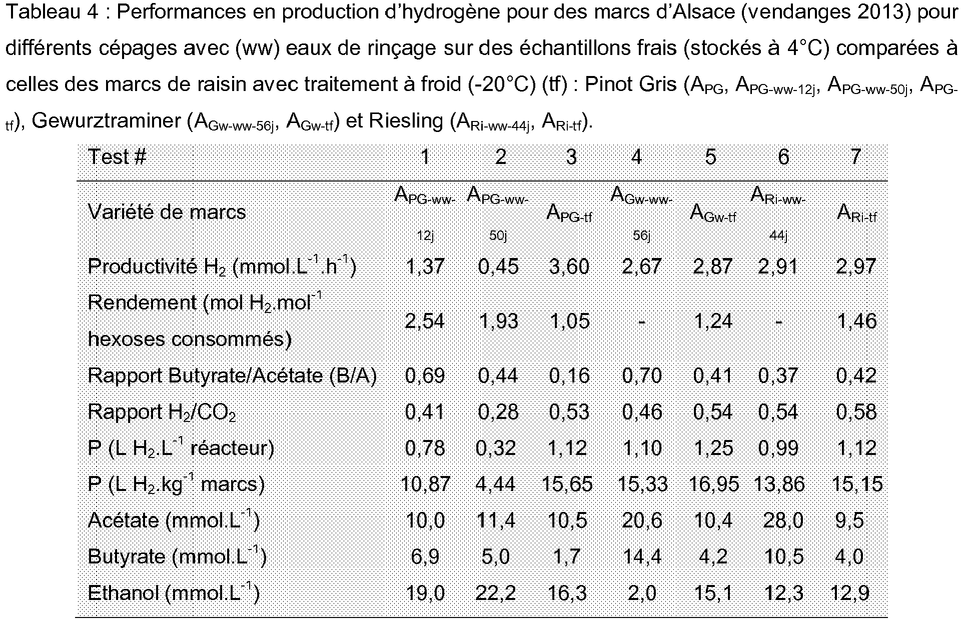

Pour les marcs de raisin issus de cépage pinot gris additionnés des eaux de rinçage, il a été remarqué que pour ces échantillons ont été obtenus les meilleurs rendements en hydrogène par rapport à l'ensemble des échantillons précédemment testés mettant en évidence que la microflore présente dans les marcs « frais » est la plus adaptée pour métaboliser les sucres de la biomasse et pour produire de l'hydrogène. Il a été également constaté que la durée du stockage à 4°C (tests 1 et 2) a un impact sur les performances de production en hydrogène ; a été observée une baisse importante de la productivité (67,1%), un rendement en hydrogène et un rapport H₂/CO₂ diminués respectivement de 24,0% et 31,7% alors que les teneurs en métabolites ont peu variées. Les analyses de gaz sur des échantillons de marcs conditionnés à 4°C ont montré qu'en effet, lors du stockage, la fermentation a lieu avec une production très majoritaire de CO₂. Pour ce même cépage, la comparaison avec la biomasse stockée à froid (-20°C) (test 3) a montré une plus forte vitesse de production en H₂ pour cet échantillon alors que le rendement a été diminué de plus de la moitié et que la production en hydrogène par litre de réacteur a été maintenue. Ces résultats ont montré que ce traitement à froid a un effet sur le consortium microbien producteur d'hydrogène. Pour les marcs de Gewurztraminer et de Riesling, malgré un stockage sur une durée de 44 et 56 jours, les performances en termes de production d'hydrogène sont bonnes avec pour le Riesling (test 6) une teneur du milieu réactionnel plus forte en acétate par rapport aux autres tests.

### EXEMPLE 4 : PERFORMANCE EN PRODUCTION D'HYDROGENE POUR LA BIOMASSE « BOURBES »

Tout comme pour la biomasse « marcs », les bourbes issues des vendanges 2013 provenant de différents cépages issus de différents terroirs: Alsace (Domaine Pfister, Dahlenheim) et Bourgogne (Domaine de Poncétys du Lycée Viticole de Macon-Davayé) ont été utilisés en tant que substrat et inoculum microbien. Les paramètres testés dans un premier temps pour les échantillons de bourbes d'Alsace sont la variation de production en fonction de la nature du cépage sur un même domaine, l'effet lié au stockage à froid (-20°C) et l'effet de la dilution des bourbes avec les eaux de rinçage de pressoir correspondantes.

Les résultats consignés dans le tableau 5 ont montré que quel que soit le cépage, sur des échantillons de bourbes « fraîches » stockées à 4°C (tests 1, 4, 7), le rendement en hydrogène a été supérieur à 1,4 mol H₂.mol⁻¹ d'hexoses avec un rapport molaire H₂/CO₂ plus élevé que pour les marcs de raisin. Le consortium microbien de la biomasse Pinot Gris a permis d'obtenir la productivité la plus élevée 10,04 mmol.L⁻¹.h⁻¹ et la biomasse Gewurztraminer a permis la production d'hydrogène la plus forte (55,00 L H₂.L⁻¹ bourbes) liée à une teneur en sucres importante ; l'analyse des métabolites a également mis en évidence un rapport molaire Butyrate/Acétate plus élevé. Comparativement aux tests réalisés sur les marcs de raisin, il a aussi été remarqué une productivité en hydrogène beaucoup plus élevée pour la biomasse « bourbes », significative de populations microbiennes particulièrement actives ou beaucoup plus concentrées.

Il est à noter que les résultats de ce travail obtenus avec des microflores endogènes sont comparables à ceux obtenus par fermentation obscure à partir de fruits mûrs mais en additionnant à la biomasse des cultures mixtes (3,8-4,5 L_{H2}.L⁻¹réacteur et 1,7-2,2 mol.mol⁻¹) [2].

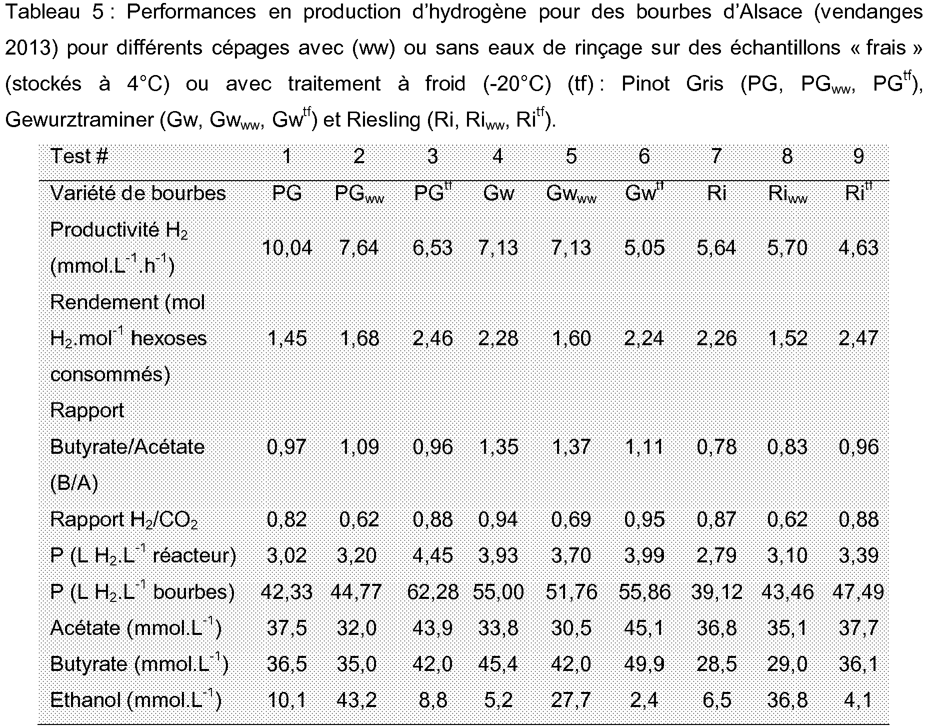

L'utilisation des eaux de rinçage servant à diluer les bourbes (tests 2, 5, 8) a mis en évidence une diminution du rendement en hydrogène (de 2,00 ± 0,47 mol.mol⁻¹ à 1,60 ± 0,08 mol.mol⁻¹) pour les 3 cépages (Pinot Gris, Gewurztraminer, Riesling) ainsi qu'un fléchissement de la productivité spécifiquement pour le cépage Pinot Gris (de 10,04 à 7,64 mmol.L⁻¹.h⁻¹). La production en hydrogène par litre de réacteur a été cependant quasi-équivalente bien que la quantité de sucres était logiquement plus élevée avec l'addition des eaux de rinçage. D'autre part, il a été remarqué que la production d'éthanol était favorisée induisant un rapport molaire H₂/CO₂ plus faible (en moyenne 0,64 et 0,88 respectivement avec et sans les eaux de rinçage). L'existence de consortia microbiens dans les eaux de rinçage entrant en compétition avec ceux présents dans les bourbes pour métaboliser les sucres a pu être envisagée.

Le traitement à froid (-20°C) de la biomasse « bourbes » (tests 3, 6, 9) a induit, par rapport à celui obtenu pour la biomasse « fraîche », une augmentation en moyenne du rendement en hydrogène (de 2,00 ± 0,47 mol.mol⁻¹ à 2,31 ± 0,13 mol.mol⁻¹) et de la production d'hydrogène pour chacun des 3 cépages testés ; les vitesses de production étant moindres.

La reproductibilité en matière de production d'hydrogène d'une année sur l'autre pour un même cépage (Pinot Gris) a été testée de même que l'effet d'un stockage à 4°C (durée de 7 jours et 35 jours) versus un stockage à -20°C (tf), la variation de production en fonction de la durée du stockage à froid au-delà de 6 mois (dans deux bioréacteurs différents) et pour les échantillons de Bourgogne, l'impact du mode de traitement de la vigne pour un même cépage (Chardonnay) et un même domaine. Les résultats de production sont consignés dans le tableau 6.

Les bourbes de Pinot Gris du même domaine prises dans un état de conservation semblable (stockées à 4°C) (tests 2, 3) ont donné lieu d'une année sur l'autre à une productivité en hydrogène équivalente, une production plus élevée en 2012 alors que le rendement en hydrogène pour le lot 2013 était presque le double de celui de 2012. L'effet positif de la conservation à -20°C (test 3, 4) sur le rendement, la productivité et la production en hydrogène pour les échantillons de vendanges 2012 a également été observé.

La conservation de la biomasse à 4°C de 7 jours à 35 jours (tests 1, 2) a eu principalement un effet sur la productivité en hydrogène, celle-ci passant de 10,04 à 3,56 mmol.L⁻¹.h⁻¹ avec une production en hydrogène plus faible liée à la consommation de sucres métabolisables lors de la conservation de l'échantillon.

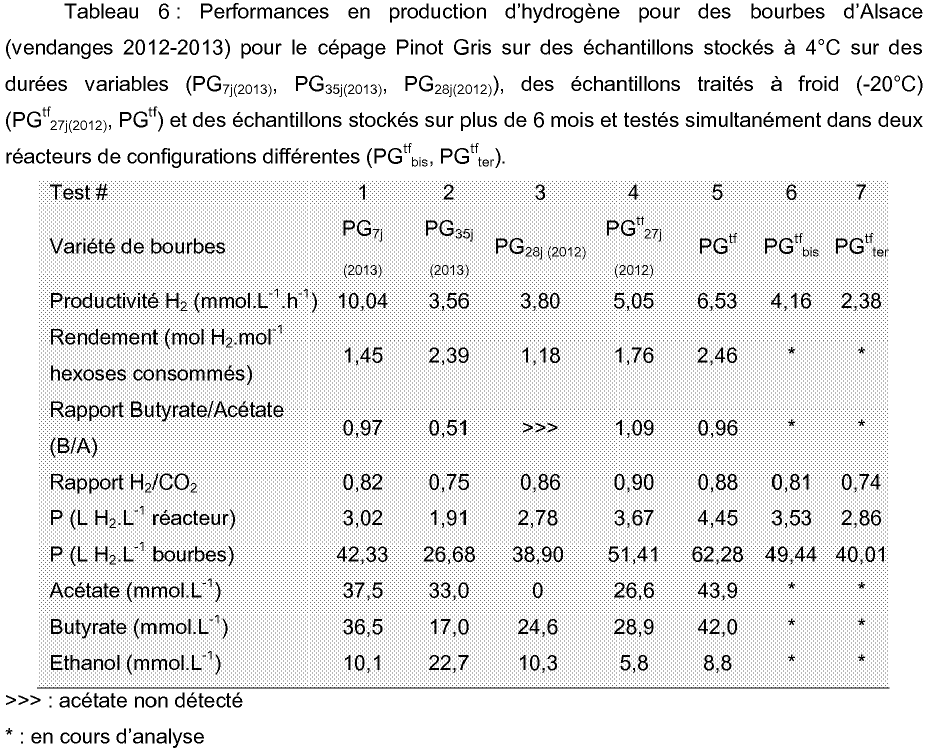

Les bourbes de Pinot Gris conservées à -20°C pendant plus de 6 mois ont été mises en oeuvre dans deux bioréacteurs différents ayant des configurations proches, l'un étant celui utilisé pour tous les essais réalisés (PG^{tf}_{bis}), l'autre nouvellement installé étant situé dans un autre bâtiment (PG^{tf}ₜₑᵣ). Les premières données (tableau 6) ont attesté du fait que la biomasse stockée plus de 6 mois à - 20°C est toujours active pour la production d'hydrogène et d'autre part, que la biomasse mise en oeuvre dans un réacteur neuf a également été active écartant ainsi toute hypothèse de contamination. La production d'hydrogène pour les tests 6, 7 a été plus faible que pour l'essai (test 5), explicable potentiellement par une quantité moindre de matières à métaboliser (réduction de la teneur en sucres) ou par un vieillissement de la biomasse (hypothèses à lever par les analyses ultérieures). Le test réalisé dans le nouveau réacteur (test 7) a mis en évidence en première approche une production d'hydrogène moins efficace ; ceci étant attribuable à un mode d'extraction des gaz non optimisé et l'existence d'une pâle de cisaillement supplémentaire dans le bioréacteur.

Les bourbes de Chardonnay de Bourgogne issues de différents modes de traitement des cultures (tableau 7) (tests 1, 3, 5) ont eu un impact plus mesuré sur le rendement en hydrogène (allant de 1,80 à 2,18 mol.mol⁻¹), que pour les marcs de raisin correspondants. Le meilleur rendement en hydrogène a été, comme pour les marcs de raisin, obtenu pour l'échantillon ayant subi le traitement éco-phytosanitaire avec le rapport molaire butyrate/acétate le plus élevé (2,32). Il est à noter pour les 3 échantillons une production par litre de bourbes très proches (50,58 ± 2,87 L H₂.L⁻¹ bourbes) et des productivités du même ordre de grandeur (3,52 ± 0,37 mmol.L⁻¹.h⁻¹).

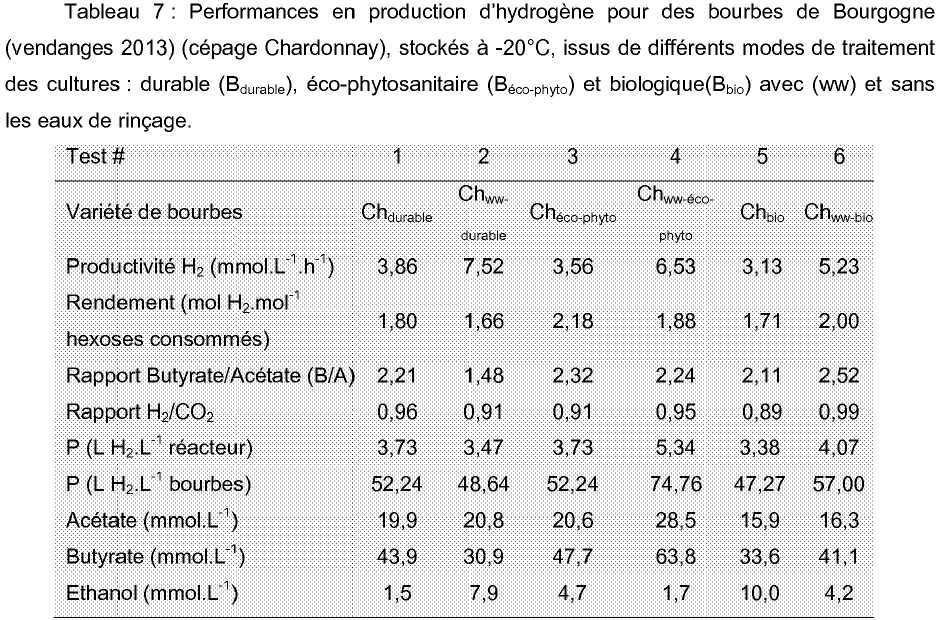

L'utilisation des eaux de rinçage additionnées aux bourbes de Bourgogne issus de divers modes de cultures de la vigne a eu pour impact d'augmenter très significativement la productivité en hydrogène (6,43 ± 1,15 mmol.L⁻¹.h⁻¹ avec eaux de rinçage). Pour les échantillons ayant subi le traitement éco-phytosanitaire et biologique, il est à noter une augmentation substantielle de la production par litre de bourbes par rapport aux tests sans eaux de rinçage, respectivement de 43.1% et 19,9%. Pour ces échantillons, il a été remarqué également un rapport H₂/CO₂ élevé, respectivement de 0,95 et 0,99.

### EXEMPLE 5 : PERFORMANCE EN PRODUCTION D'HYDROGENE POUR LA BIOMASSE « EAUX DE RINÇAGE »

Les résultats exposés dans le tableau 8 montrent que les eaux de rinçage d'Alsace (cépage Pinot Gris et Gewurztraminer) peu riches en matières métabolisables (teneur en hexose très faible) (tests 1 et 2) présentent une activité en production d'hydrogène très faible avec un rapport molaire H₂/CO₂ très en faveur de la production de dioxyde de carbone, significative d'une production par solvantogénèse.

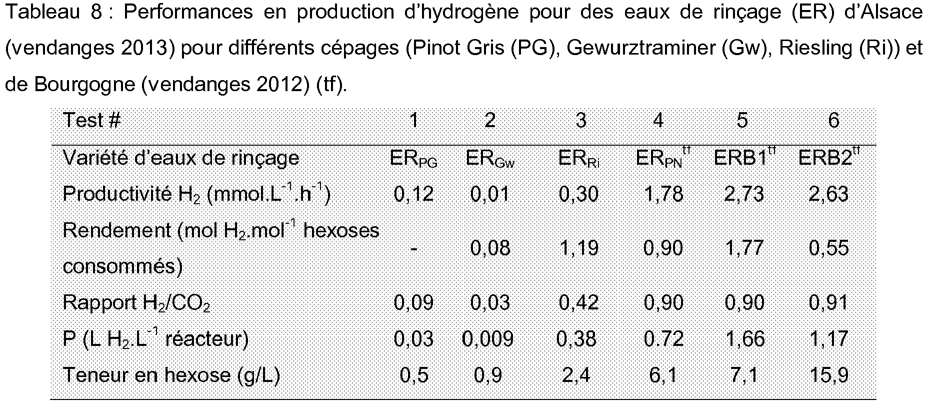

Lorsque les eaux de rinçage étaient plus concentrées en sucres (tests 4, 5 et 6) et conditionnées à froid (-20°C), le rendement en hydrogène, certes en général inférieur à celui obtenu sur les divers échantillons de marcs de raisin dilués et d'autant plus sur les bourbes diluées, a révélé l'activité d'un consortium microbien endogène favorable à la production d'hydrogène ; le rapport H₂/CO₂ étant de 0,90.

Ces résultats ont mis en évidence que substrat et consortium microbien sont également présents dans les eaux de rinçage et cela a suggéré la nécessité de récupérer les eaux de premier rinçage des pressoirs afin de concentrer au maximum en matières métabolisables la biomasse à traiter.

### EXEMPLE 6 : PERFORMANCE EN PRODUCTION D'HYDROGENE POUR LES BIOMASSES « GATEAU DE FILTRATION » ET « LIES »

Les résultats exposés dans le tableau 9 montrent les performances en production d'hydrogène obtenus pour différentes biomasses issues du traitement vitivinicole : bourbes (B), gâteau de filtration (G) et lies de cuve de vinification (L) d'un même domaine (Alsace) et pour un même cépage (Riesling).

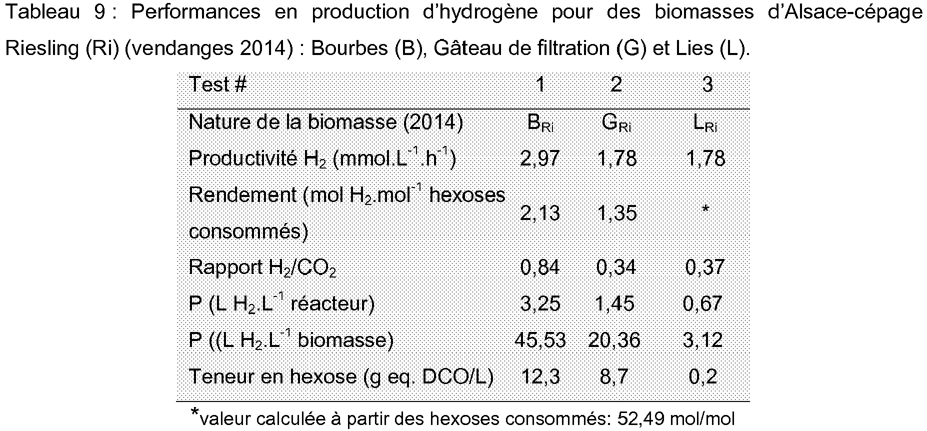

Conformément aux attentes, la biomasse la plus riche en hexose (bourbes) permet d'atteindre la production volumique en hydrogène la plus importante avec une productivité élevée.

Cependant, il est à noter que les lies bien que très peu riches en sucres permettent d'atteindre une production en hydrogène par litre de réacteur certes 2 fois moindre par rapport au gâteau de filtration mais avec une teneur en hexose 43 fois moins élevée et une vitesse de production en hydrogène similaire.

Affichant un rendement en hydrogène atypique, la biomasse 'lies' contient donc de la matière fermentescible (autres que les hexoses) en hydrogène.

Bien que le rapport molaire H₂/CO₂ soit plus faible, le rendement en hydrogène obtenu pour le gâteau de filtration est équivalent à celui obtenu pour les marcs de raisin bien que le rapport molaire H₂/CO₂ soit plus faible.

Ainsi, cette étude montre que la valorisation du gâteau de filtration et des lies est également possible pour la production d'hydrogène.

### EXEMPLE 7: STRUCTURE DES COMMUNAUTES MICROBIENNES ET EVOLUTION AU COURS DE LA FERMENTATION

Une étude de l'évolution des communautés microbiennes en cours de fermentation a été menée sur un échantillon de biomasse bourbes Pinot gris d'Alsace (vendanges 2014) stockée à -20°C pendant 47 jours (ptf 47 jours).

Le séquençage a été réalisé en ciblant les régions variables V4-V5 des séquences de l'ARN ribosomique 16S des procaryotes. Les séquences partageant au minimum 97% de similarité ont été groupées en OTU (unités taxonomiques opérationnelles) à l'aide du logiciel Mothur. Ce pourcentage correspond à un pourcentage intermédiaire entre les genres (96 %) et les espèces (98 % pour la plupart des genres bactériens sauf le genre *Bacillus* > 98 %).

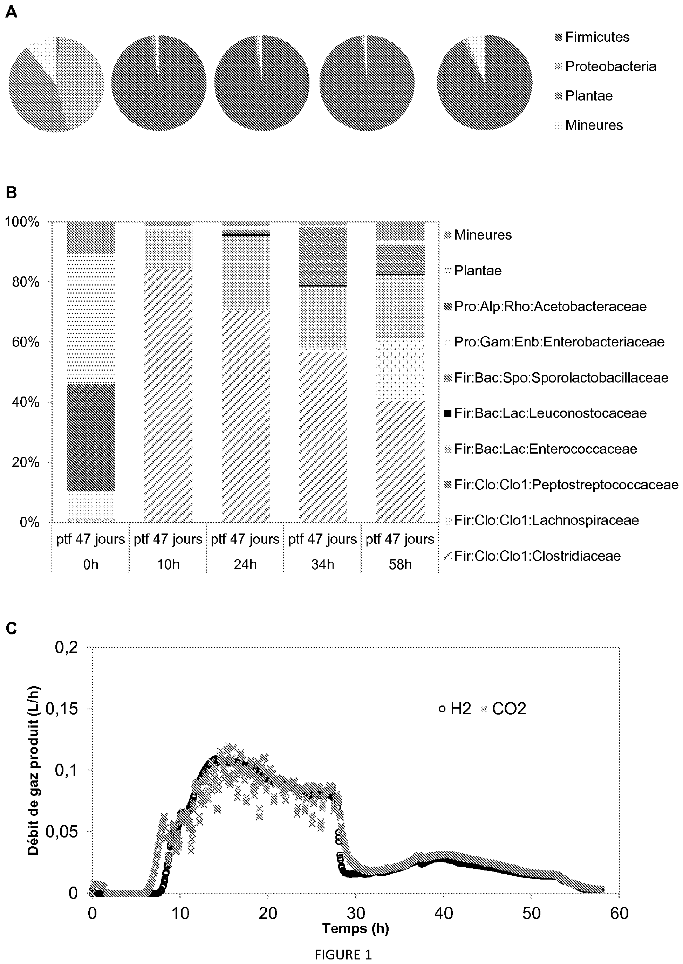

La taxonomie aux niveaux du phylum et de la famille des échantillons prélevés est représentée sur les figures 1a et 1b en fonction de la durée de fermentation et mise en parallèle avec la production d'hydrogène (figure 1c).

A l'état initial, *Proteobacteria* est le phylum bactérien le plus abondant (45,1%). Un OTU ayant une abondance relative de 45,3% a été affilié au règne *Plantae* (figure 1a). La comparaison des séquences représentatives des OTU avec la base de données environnementale (nr_nt) de NCBI grâce au logiciel en ligne BLAST, a permis d'affilier ces séquences à l'espèce *Vitis vinifera* du règne *Plantae* avec 100,0 % de similarité. Ainsi, à l'état initial, la biomasse 'bourbes' testée est constituée principalement de bactéries appartenant au phylum *Proteobacteria.*

Au cours de la fermentation, le phylum *Firmicutes* devient le plus abondant au détriment du phylum *Proteobacteria.* En effet, les valeurs d'abondances relatives du phylum *Firmicutes* passent de 1,0% initialement à des valeurs comprises entre 92,3% (58 h) et 97,4% (10 h) ; et celles du phylum *Proteobacteria* passent de 45,1% (0 h) à des valeurs comprises entre 0,6% (34 h) et 1,8% (58 h). Les conditions dans lesquelles la fermentation obscure est pratiquée permettent de « favoriser » les bactéries appartenant au phylum *Firmicutes.*

Même si le phylum *Firmicutes* reste le plus abondant, la classification taxonomique des séquences au niveau du taxon évolue au cours de la fermentation (figure 1b). En effet, initialement, la famille *Acetobacteriaceae* (phylum *Proteobacteria*) est la plus abondante après *Vitis vinifera* (35,5 %), puis à 10 h de fermentation, la famille *Clostridiaceae* devient la plus abondante (84,3%) et l'abondance relative de la famille *Enterococcaceae* augmente en passant de 0,3 % à 12,9 %. Ces deux familles restent les principales jusqu'à la fin de fermentation (58 h) mais avec des abondances relatives différentes. Au cours du temps, l'abondance relative des *Clostridiaceae* diminue de moitié en passant de 84,3% (10 h) à 40,5% (58 h) alors que celle des *Enterococcaceae* augmente en passant de 12,9% (10 h) à 20,7% (58 h). A 34 h une autre famille devient également majoritaire avec 19,3% d'abondance relative, les *Sporolactobacillaceae.* L'abondance de cette famille diminue de moitié en fin de fermentation (9,7%) en faveur de la famille *Lachnospiraceae* qui passe de 1,4% (34 h) à une abondance relative de 21,0% (58 h).

### EXEMPLE 8 : PRODUCTION D'HYDROGENE ET STRUCTURE DES COMMUNAUTES MICROBIENNES EN FONCTION DE LA DUREE DE STOCKAGE A -20°C DE LA BIOMASSE

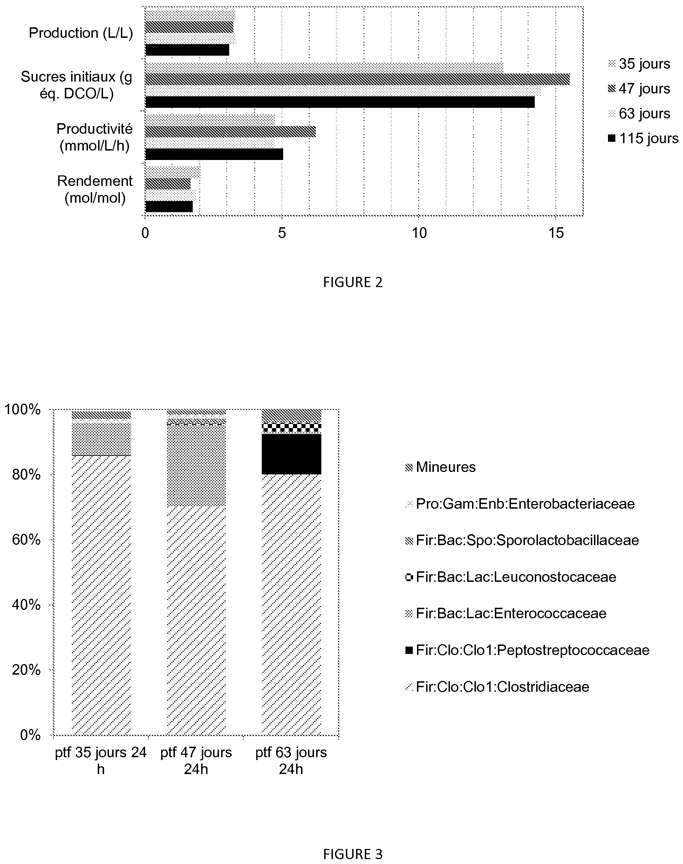

Les valeurs de performances de production d'hydrogène obtenues en fonction de la durée de stockage à -20°C de la biomasse bourbes d'Alsace Pinot Gris (vendanges 2014) sont représentées sur la figure 2.

Les valeurs de productivités obtenues à partir des différents tests de fermentation varient entre 4,75 et 6,24 mmol/L/h. La valeur de productivité obtenue lors du test '47 jours' (6,24 mmol/L/h) est la plus élevée, en partie due à la concentration initiale en sucres plus élevée pour ce test (15,5 g équivalent DCO/L) que pour les autres tests.

Les valeurs de rendement en hydrogène obtenues en fonction des différentes durées de stockage de la biomasse à -20°C varient entre 1,67 et 2,02 mol/mol, soit une variation de 9%.

Il est donc observé que la durée du stockage a un impact limité sur la production d'hydrogène permettant d'envisager le stockage de la biomasse sur une durée importante ; les variations observées sur les performances de production sont potentiellement attribuables à la diversité microbienne et par conséquent, les voies métaboliques utilisées par la microflore endogène.

La figure 3 représente les classifications taxonomiques et les indices de diversité microbienne des échantillons prélevés à 24 h de fermentation lorsque la biomasse a été stockée pendant 35, 47 et 63 jours. Comme remarqué précédemment, le phylum *Firmicutes* est le plus abondant avec des valeurs d'abondances relatives variant entre 96,1% et 97,4% respectivement pour '35 jours' et '47 jours' (figure non représentée).

Au niveau des familles (figure 3), les tests '35 jours' et '47 jours' ont une distribution similaire avec la famille majoritaire *Clostridiaceae* avec 85,8% et 84,3% d'abondance relative respective, et la famille *Enterococcaceae* est la deuxième famille la plus abondante (respectivement 9,7% et 12,9%). En revanche, les familles *Clostridiaceae* et *Peptostreptococcaceae* sont les plus abondantes lors du test '63 jours', avec respectivement 80,1% et 12,4%.

L'affiliation des séquences à l'espèce ou au genre microbien la plus proche est présentée dans le tableau 10 pour les tests à 35 jours, 47 jours et 63 jours de stockage de la biomasse à -20°C.

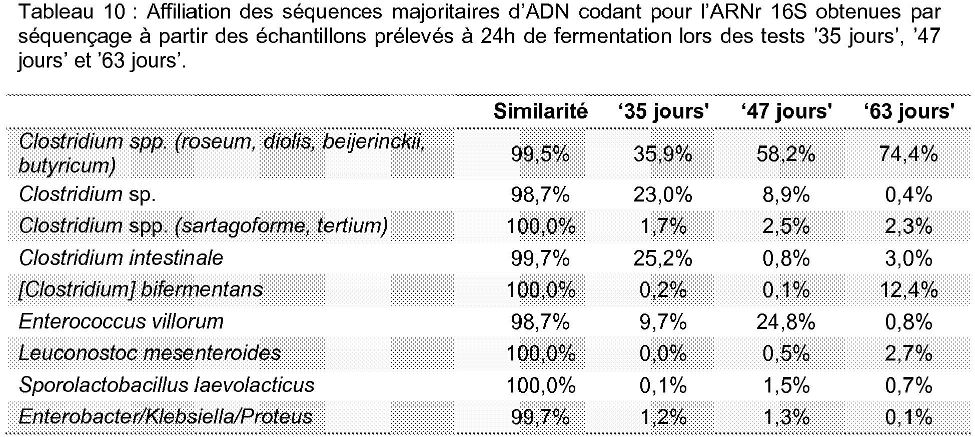

Le genre affilié à la séquence représentative de l'OTU dominant dans tous les tests, est *Clostridium* spp. (*roseum, diolis, beijrinckii, butyricum*) avec des abondances relatives de 35,9%, 80,1% et 74,4%, respectivement pour les tests '35 jours', '47 jours' et '63 jours'. Seul le test '35 jours' contient deux OTU sous-dominants affiliés à *Clostridium intestinale* et *Clostridium* sp. avec respectivement 25,2% et 23,0% d'abondance relative. Ces deux espèces sont minoritaires dans les tests '47 jours' et '63 jours' (abondances relatives inférieures à 3%). Les espèces intermédiaires affiliées aux autres séquences sont : *Enterococcus villorum* avec des abondances relatives de 9,7% et 12,9% respectivement pour le test '35 jours' et '47 jours' ; *[Clostridium] bifermentans* avec une abondance relative de 12,4% pour le test '63 jours'. Les phylotypes bactériens minoritaires présents dans tous les tests sont principalement *Clostridium* spp. (*sartagoforme, tertium*), *Leuconostoc mesenteroides* et les phylotypes bactériens entériques appartenant à la famille des *Enterobacteriaceae* avec des abondances relatives inférieures à 2,7%.

En conclusion, plus la durée de stockage est longue et plus *Clostridium* spp. *(roseum, diolis, beijrinckii*, *butyricum)* est majoritaire, ce qui signifie que cette bactérie est résistante à la congélation jusqu'à 63 jours de stockage contrairement à *Clostridium* sp., impactée par la durée de congélation. Les données de performances ont montré que la production d'hydrogène n'est pas affectée par la durée de stockage, bien que les abondances relatives de ces espèces soient différentes. Ainsi, *Clostridium* spp. (*roseum, diolis, beijrinckii*, *butyricum*) ne sont pas les seuls types de bactéries responsables de la production d'hydrogène. En effet, *Clostridium intestinale* et plusieurs souches qui n'ont pas été formellement caractérisées taxonomiquement (*Clostridium* sp.) sont capables de produire de l'hydrogène. *[Clostridium] bifermentans* présente dans le test '63 jours' fermente à la fois les sucres et les acides aminés et a la particularité de produire majoritairement de l'hydrogène via les voies acétate et formiate.

### EXEMPLE 9: INFLUENCE DE LA TENEUR DE LA CHARGE POUR LA BIOMASSE « BOURBES » SUR LA PRODUCTION D'HYDROGENE ET SUR LA DIVERSITE MICROBIENNE

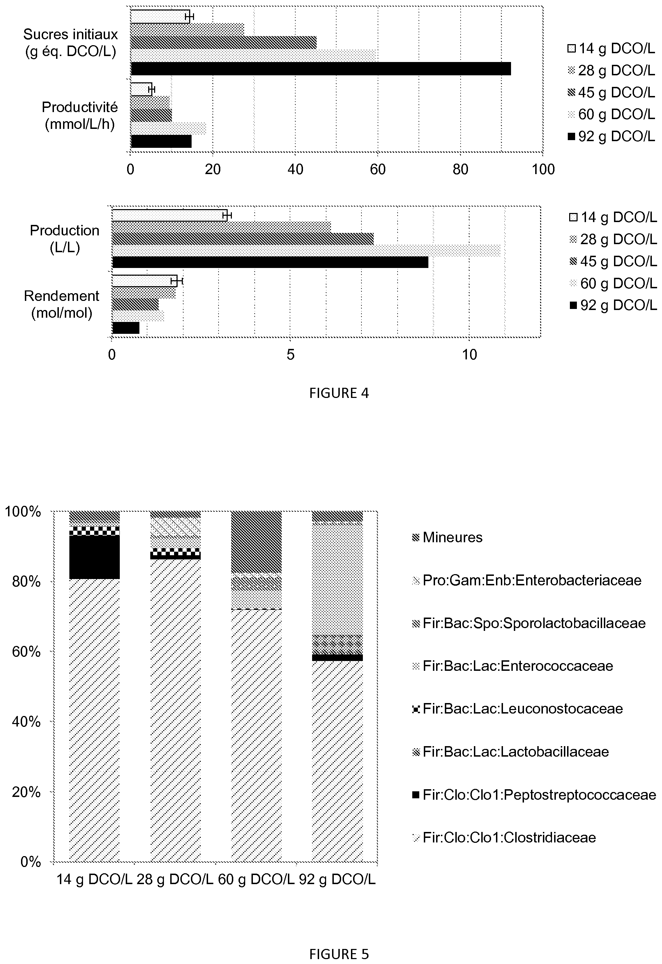

Les performances de production d'hydrogène obtenues pour la biomasse Bourbes Pinot Gris d'Alsace (vendanges 2014) en fonction de la charge mise en oeuvre dans le bioréacteur, de 14 g DCO/L à 92g DCO/L, sont représentées sur la figure 4.

Plus la charge est élevée, plus les volumes d'hydrogène produits sont importantes et plus les valeurs de productivités obtenues sont élevées jusqu'à une teneur de 60 g DCO/L (5,20 ± 0,71 mmol/L/h pour le test à 14 g DCO/L et 18,41 mmol/L/h pour le test à 60 g DCO/L). Ces résultats montrent bien que la biomasse est non seulement source de matières fermentescibles mais aussi source de microorganismes producteurs d'hydrogène. Pour une teneur de 92 g DCO/L, la productivité obtenue (14,85 mmol/L/h) est inférieure au test à 60 g DCO/L.

Jusqu'à 28 g DCO/L, la valeur de rendement en hydrogène n'est pas impactée par la charge (1,79 mol/mol obtenu pour '28 g DCO/L' contre 1,82 ± 0,16 mol/mol pour le test '14 g DCO/L'). Au-delà de 28 g DCO/L et jusqu'à 60 g DCO/L, les valeurs de rendements fléchissent légèrement (1,31 et 1,47 mol/mol, respectivement pour les tests '45 g DCO/L' et '60 g DCO/L') et de façon plus forte pour une teneur de 92 g DCO/L (0,77 mol/mol). Ainsi, une charge trop élevée en sucres impacte la production et la vitesse de production en hydrogène soit par l'inhibition partielle de certaines bactéries productrices d'hydrogène, soit par le fait que les biocatalyseurs ont atteint leur vitesse maximale de conversion des sucres en hydrogène.

L'analyse de la structure microbienne par la classification taxonomique au niveau des familles de séquences obtenues pour chaque échantillon (figure 5) montre que, parmi le phylum *Firmicutes*, phylum majoritaire, *Clostridiaceae* est la famille la plus abondante à 24 h de fermentation quelle que soit la charge avec des valeurs d'abondances relatives comprises entre 57,4% ('92 g DCO/L') et 86,4% ('28 g DCO/L').

Il est à noter que plus la charge est élevée et plus l'abondance relative à cette famille est faible. Les autres familles présentes, plus minoritaires, sont principalement: *Peptostreptococcaceae* (12,4%) pour le test '14 g DCO/L'; *Enterobacteriaceae* (5,2%) pour le test '28 g DCO/L' ; *Enterococcaceae* pour les tests '60 g DCO/L' et '92 g DCO/L'avec respectivement 5,2% et 31,2% d'abondances relatives. L'abondance relative de cette dernière famille semble corréler avec la charge. En effet, plus la charge augmente et plus l'abondance relative des *Enterococcaceae* augmente également.

L'affiliation des séquences à l'espèce ou au genre microbien le plus proche est présentée dans le tableau 11.

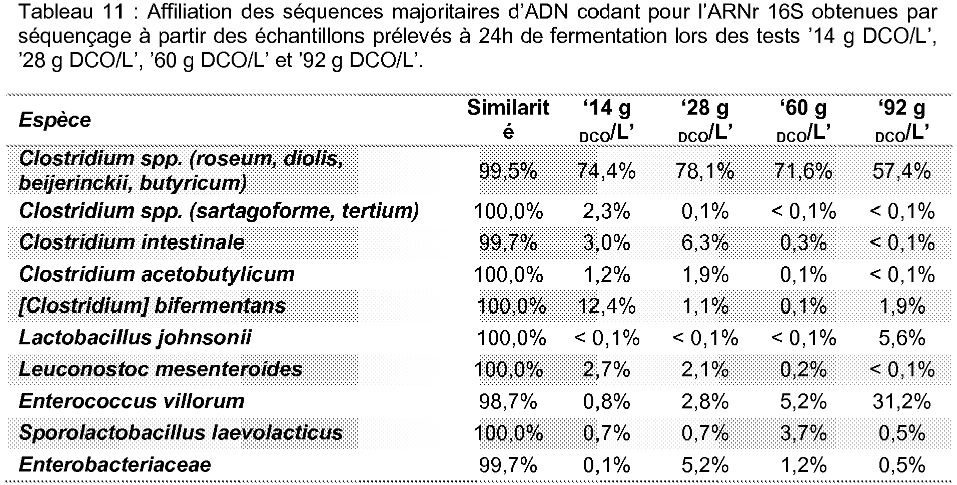

Le genre dominant affilié à l'OTU majoritaire est *Clostridium* spp. (*roseum, diolis*, *beijrinckii*, *butyricum*) quelle que soit la charge avec des abondances relatives similaires les plus élevées pour une charge jusqu'à 60 g DCO/L et une abondance plus faible pour le test '92 g DCO/L'.

Par ailleurs, on observe que plus la charge en biomasse est élevée et plus l'abondance relative de l'espèce *Enterococcus villorum* est élevée (0,8 % à 14 g DCO/L contre 31,2 % à 92 g DCO/L). Il semble donc possible que le fléchissement en production d'hydrogène à une teneur de 92 g DCO/L puisse être lié à l'inhibition partielle des bactéries productrices d'hydrogène (*Clostridium* spp.).

D'autre part, les espèces minoritaires affiliées à chaque test sont : *[Clostridium] bifermentans* (12,4%) pour le test '14 g DCO/L', *Clostridium intestinale* (6,3%) et *Enterobacteriaceae* (5,2%) pour le test '28 g DCO/L', *Enterococcus villorum* pour le test '60 g DCO/L' (5,2%) et *Lactobacillus johnsonii* (31,2%) pour le test '92 g DCO/L'. Trois espèces affiliées à trois OTU minoritaires (< 3,7%) à très minoritaires (< 0,1%) sont présentes dans tous les tests: *Clostridium spp. (sartagoforme, tertium), Clostridium acetobutylicum, Leuconostoc mesenteroides et Sporolactobacillus laevolacticus.* Ainsi, le séquençage permet de mettre en évidence que plus la teneur en biomasse et la quantité initiale de sucres sont élevées plus la diversité microbienne est importante.

### EXEMPLE 10 : INFLUENCE DE L'ETAT DE CONSERVATION DE LA BIOMASSE « BOURBES » SUR LA PRODUCTION D'HYDROGENE ET SUR LA DIVERSITE MICROBIENNE - INFLUENCE DE LA NATURE DU GAZ DE BALAYAGE PERMETTANT L'EXTRACTION DES GAZ PRODUTS

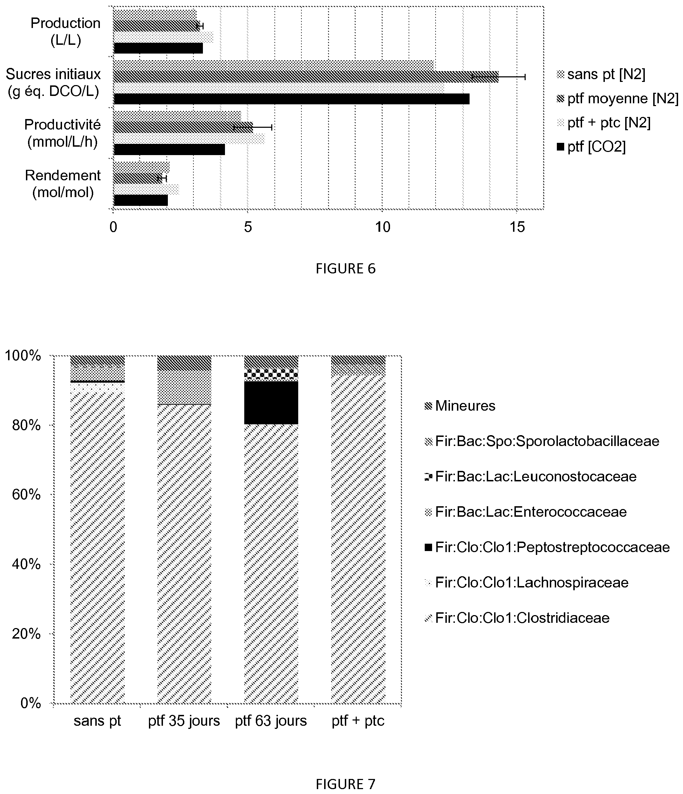

Dans cette étude, les performances en production d'hydrogène (figure 6) de la biomasse bourbes d'Alsace, Pinot Gris (vendanges 2014) stockée à 4 °C ('sans pt') est comparée à celle de la biomasse stockée à -20°C ('ptf') et à la biomasse stockée à -20°C et ayant subi un prétraitement thermique à 70°C (pendant 1h) ('ptfc'). Parallèlement, une étude comparative sur l'impact de la nature du gaz de balayage a été réalisée sur la biomasse stockée à 20°C.

Les valeurs de production en hydrogène obtenues pour les tests 'sans ptf', 'ptf (moyenne effectuée sur les tests entre 35 et 115 jours de stockage) et 'ptf + CO2' sont proches avec, pour le test où la biomasse stockée à -20°C a subi un prétraitement thermique à 70°C, un volume d'hydrogène produit le plus élevé (3,7 L/L).

Les valeurs de productivités en hydrogène obtenues à partir des différents tests varient entre 4,16 et 5,64 mmol/L/h. Les valeurs obtenues pour les tests 'ptf' sont statistiquement supérieures aux tests 'sans ptf' et 'ptf + CO2', seule 5,64 mmol/L/h obtenue lors du test 'ptf + ptc' est plus élevée.

Les valeurs de rendements obtenues à partir des différents tests varient entre 1,82 ± 0,16 et 2,45 mol/mol. La valeur de rendement obtenue pour le test où la biomasse congelée a été prétraitée thermiquement est la plus élevée et la plus proche du rendement maximal théorique *in vivo* (61 %).

De plus, on peut noter que l'utilisation du CO₂ comme gaz de balayage n'impacte pas les performances de production d'hydrogène (rendement) comparativement à l'utilisation de l'azote et permet d'envisager le recyclage du dioxyde de carbone produit réduisant ainsi la consommation de gaz externe.

L'étude réalisée sur l'influence de la durée de stockage a permis de mettre en évidence une évolution de la flore microbienne en fonction de la durée de stockage. C'est pourquoi, deux tests 'ptf 35 jours' et 'ptf 63 jours' étant les plus proches en termes de durées de stockage, ont été choisi pour comparer respectivement les tests 'sans pt' (12 jours) et 'ptf + ptc' (154 jours)

La figure 7 représente la classification taxonomique au niveau de la famille obtenue pour les échantillons prétraités ou non.

Le phylum *Firmicutes* est le plus abondant avec des valeurs d'abondances relatives variant entre 96,0 % et 97,6 % respectivement pour '35 jours' et 'ptf + ptc' (figure non représentée). *Clostridiaceae* est la famille la plus abondante quel que soit le test avec des valeurs d'abondances relatives variant de 80,3% et 94,5%. Contrairement aux tests 'ptf 35 jours' et 'ptf 63 jours', les tests 'sans pt' et 'ptf + ptc' sont composés de familles minoritaires ayant des abondances relatives inférieures à 3,8%.

L'affiliation des séquences à l'espèce ou au genre microbien le plus proche est présentée dans le tableau 12 pour les tests 'sans pt' et 'ptf + ptc'.

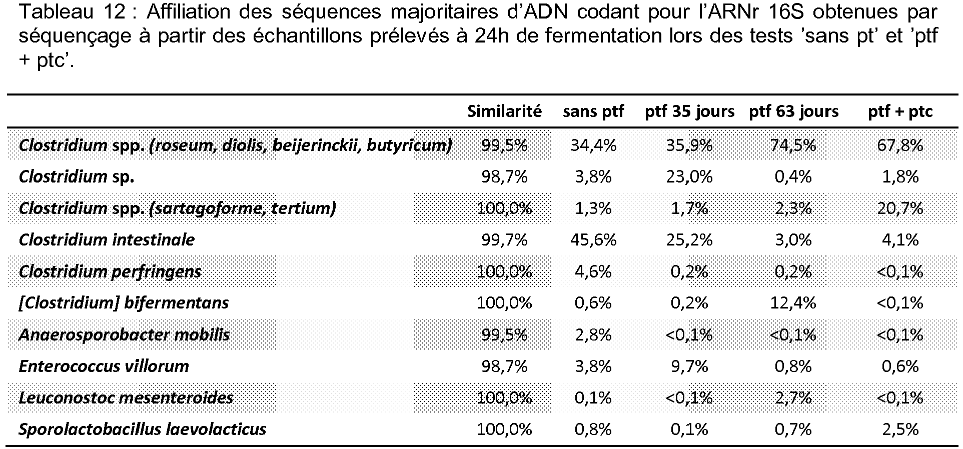

Lorsque la biomasse n'a pas été prétraitée, une espèce dominante est affiliée à l'OTU majoritaire : *Clostridium intestinale* (45,6 %) alors que dans l'étude précédente sur la durée de stockage de la biomasse à -20°C, *Clostridium* spp. (*roseum, diolis, beijrinckii, butyricum*) était l'espèce majoritaire. Dans le cas du test 'sans pt', cette espèce est « seulement » co-dominante avec 34,4 % d'abondance relative. Le stockage de la biomasse à -20°C affecte l'espèce *Clostridium intestinale* dont l'abondance relative a presque diminué de moitié passant de 45,6% à 25,2%, en faveur de souches de genre *Clostridium* qui lorsque la biomasse est stockée à -20°C pendant 35 jours passe de 3,8% à 23,0%. *Clostridium perfringens* et *Anaerosporobacter mobilis* sont aussi être affectées par le prétraitement à froid, leurs abondances relatives respectives passent de 4,6% à 0,2%, et de 2,8% à < 0,1%. En revanche, *Enterococcus villorum* n'est pas impactée par le prétraitement à froid car elle est présente dans les deux tests (respectivement de 3,8% et 9,7%). Quant au prétraitement à chaud, il affecte négativement *[Clostridium] bifermentans* et *Leuconostoc mesenteroides,* pour lesquelles les abondances relatives passent respectivement de 12,4% à < 0,1%, et de 2,7% à < 0,1%. En revanche, le prétraitement thermique chaud favorise *Clostridium* spp. (*sartagoforme*, *tertium*) dont l'abondance relative passe de 2,3% à 20,7%.

### EXEMPLE 11 : STRUCTURE DES COMMUNAUTES MICROBIENNES DE LA BIOMASSE « MARCS DE RAISIN »

L'étude de la structure des communautés microbiennes sur des échantillons prélevés en cours de fermentation (24h) a été réalisée pour des marcs de raisin issus des vendanges 2013, stockés à -20°C, et provenant de différents terroirs : Alsace (cépages Pinot Gris (A_{PG}) et Muscat (A_{M})) et Bourgogne (cépage Chardonnay (traitement de culture éco-phytosanitaire (B_{éco-phyto}), dont les performances en production d'hydrogène sont consignées dans le tableau 3.

L'affiliation des séquences à l'espèce ou au genre microbien la plus proche est présentée dans le tableau 13 pour ces 3 échantillons.

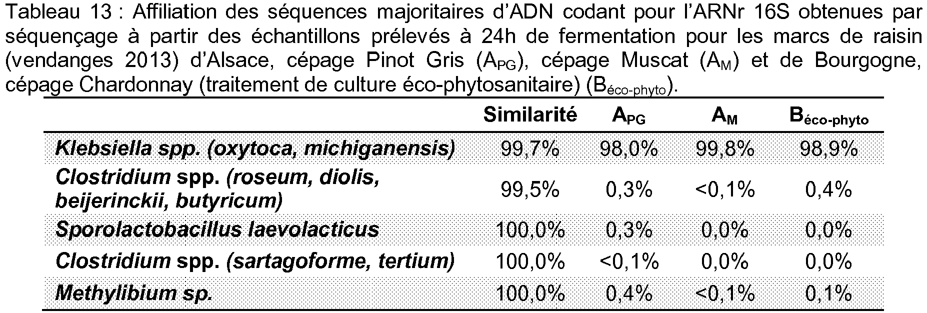

Pour les 3 échantillons de marcs, le phylum *Proteobacteria* est le plus abondant avec des valeurs d'abondance relatives de 98,4%, 99,9% et 99,0% respectivement pour les échantillons A_{PG}, A_{M} et B_{éco-phyto}. L'espèce dominante est affiliée à l'OTU majoritaire : *Klebsiella spp. (oxytoca, michiganensis)* (98,0%, 99,8% et 98,9% respectivement pour les échantillons A_{PG}, A_{M} et B_{éco-phyto}).

Ces résultats montrent que la microflore endogène présente dans les marcs en cours de fermentation est différente de celle présentes dans la biomasse bourbes pour lesquelles le phylum *Firmicutes* est le plus abondant.

### En résumé :

La fermentation selon le procédé de l'invention a été réalisée dans un réacteur de 1L à double enveloppe placé à l'obscurité et doté d'un système d'extraction des gaz mis au point au laboratoire permettant à la fois d'assurer les conditions d'anaérobiose et de réduire au minimum la pression partielle en hydrogène, celui-ci étant inhibiteur de l'activité microbienne. A titre indicatif, pour les bourbes, le rendement moyen en hydrogène obtenu est de 2,0 ± 0,4 mol d'H₂/mol hexose avec un volume produit compris entre 1,9 et 5,3 L H₂/L_{réacteur} selon le conditionnement de la biomasse, correspondant potentiellement à une production allant de 26,7 à 74,8 L H₂/L_{biomasse}. Pour les marcs, le rendement moyen en hydrogène s'élève à 1,3 ± 0,2 mol d'H₂/mol hexose et une production moyenne de 22,4±4,6 L H₂/kg_{biomasse}. Les biomasses ont été testées de façon combinée (bourbes + eaux de rinçage et marcs + eaux de rinçage).

Les études sur l'identification bactérienne par séquençage mettent en évidence la présence des genres *Clostridium* et *Enterobacter.*

La reproductibilité a été testée sur des bourbes issues des vendanges 2012 et 2013, les profils de production sont similaires sur des lots congelés, les volumes d'hydrogène produit et les rendements sont du même ordre de grandeur.

Les tests de la biomasse marcs avec ou sans rafles donnent lieu à des profils de production très similaires (volume d'hydrogène produit: 1,55 L (avec rafles) et 1,48 L (sans rafles).

Le consortium microbien étant composé principalement de bactéries des genres *Clostridium* ou *Enterobacter*, l'activité de la charge microbienne à l'issue de la réaction doit être traitée de la même manière que les digestats après méthanisation. C'est pourquoi le procédé de fermentation obscure pourra à terme être employé en aval de toutes opérations d'extraction de molécules d'intérêt de la biomasse initiale afin de conserver les propriétés de ces molécules.

Le passage à l'échelle industrielle peut être effectué avec le savoir-faire industriel en matière de bio digesteur pour la méthanisation. En effet, les deux procédés sont proches technologiquement.

### Listes des références

[1] Organisation Internationale de la vigne et du vin, Rapport statistique sur la vitiviniculture mondiale, 2013
[2] Christ et al., Journal of Cleaner Production, 53 : 232-242, 2013
[3] Organisation pour l'Alimentation et l'Agriculture. FAOSTAT database. http://faostat.fao.org, 2013
[4] Toscano et al., Biomass and Bioenergy, 55 : 260-267, 2013
[5] Institut Français de la Vigne et du Vin : Expérimentation nationale dur la valorisation des sous-produits vinicoles. Itinéraires 25, 2013
[6] Muradov et al., International Journal of Hydrogen Energy, 33 : 6804-6839, 2008
[7] Guo et al., International Journal of Hydrogen Energy, 35 : 10660-10673, 2010
[8] Hawkes et al., International Journal of Hydrogen Energy, 27 : 1339-1347, 2002
[9] Thauer et al., Bacteriol. Rev., 41 : 100-180, 1977
[10] Hawkes et al., International Journal of Hydrogen Energy, 32 : 172-184, 2007
[11] Rapport au nom de l'Office parlementaire d'évaluation de choix scientifiques et technologiques de MM. Laurent Kalinowski et Jean-Marc Pastor, L'hydrogène : vecteur de la transition énergétique ?, Rapport enregistré à la Présidence de l'Assemblée Nationale sous le n°1672 et à la Présidence du Sénat sous le n°253, le 19/12/13
[12] Hwang et al., Bioresource Technology, 102(2): 1051-1058, 2011
[13] Doi et al., International Journal of Hydrogen Energy, 35: 7369-7376, 2010

## Revendications

1. Procédé de production d'hydrogène comprenant la fermentation obscure de biomasses vitivinicoles, sans ajout de consortium microbien et sans prétraitement thermique, et dans lequel le gaz produit par ladite fermentation de la biomasse est extrait.

2. Procédé selon la revendication 1, dans lequel aucun apport nutritionnel additionnel n'est apporté à la biomasse.

3. Procédé selon la revendication 1 ou 2, dans lequel la biomasse vitivinicole est choisie parmi les bourbes de raisin, marcs de raisin, gâteaux de filtration, lies de vin, marcs épuisés, eaux de rinçage des matériels de récolte, de tri et de vinification.

4. Procédé selon l'une quelconque des revendications 1 à 3, dans lequel la biomasse utilisée est composée d'une ou plusieurs sources de biomasses vitivinicoles différentes.

5. Procédé selon l'une quelconque des revendications de 1 à 4, dans lequel la biomasse est issue de la filière vitivinicole et concerne les raisins utilisés pour la vinification par pressurage de la vendange, quel que soit le terroir, la couleur du raisin et le cépage.

6. Procédé selon l'une quelconque des revendications 1 à 5, dans lequel la biomasse vitivinicole est choisie parmi les bourbes ou marcs de raisin d'Alsace ou de Bourgogne, et/ou eaux de rinçage des pressoirs.

7. Procédé selon la revendication 6, dans lequel le raisin d'Alsace provient de cépages de Muscat, Auxerrois, Pinot gris, Gewurztraminer, Riesling et le raisin de Bourgogne provient de cépages de Pinot noir, Chardonnay, Pinot noir/chardonnay ou Aligoté.

8. Procédé selon l'une quelconque des revendications 1 à 7, dans lequel la fermentation est réalisée à 37°C ± 2°C.

9. Procédé selon l'une quelconque des revendications 1 à 8, dans lequel la biomasse vitivinicole est conservée par réfrigération ou congélation avant utilisation.

10. Procédé selon l'une quelconque des revendications 1 à 9, dans lequel le pH de la biomasse est supérieur à 5,5 ± 0,2 pendant la fermentation.

11. Procédé selon l'une quelconque des revendications 1 à 10, dans lequel le gaz produit par la fermentation de la biomasse est extrait par un gaz de balayage.

12. Procédé selon l'une quelconque des revendications 1 à 11, comprenant en outre une étape d'isolement du consortium microbien contenu dans la biomasse fermentée ou en fermentation.

13. Procédé pour obtenir le consortium microbien présent dans la biomasse fermentée selon le procédé de la revendication 12.

## Patentansprüche

1. Verfahren zur Herstellung von Wasserstoff, umfassend die Dunkelfermentation von Weinbaubiomassen ohne Zusatz einer Mikrobenpopulation und ohne thermische Vorbehandlung und wobei das durch die Fermentation der Biomasse produzierte Gas entnommen wird.

2. Verfahren nach Anspruch 1, wobei keinerlei zusätzlicher Nährstoffeintrag in die Biomasse eingebracht wird.

3. Verfahren nach Anspruch 1 oder 2, wobei die Weinbaubiomasse aus Traubentrub, Traubentrester, Filterkuchen, Weinhefe, verbrauchtem Trester, Spülwässern von Ernte-, Sortier- und Weinbereitungsgut ausgewählt wird.

4. Verfahren nach einem der Ansprüche 1 bis 3, wobei die eingesetzte Biomasse aus einer oder mehreren verschiedenen Quellen von Weinbaubiomassen besteht.

5. Verfahren nach einem der Ansprüche 1 bis 4, wobei die Biomasse aus dem Weinbau stammt und die für die Weinbereitung mittels Pressen der Ernte verwendeten Trauben, ungeachtet des Anbaugebiets, der Farbe der Traube und der Rebsorte, betrifft.

6. Verfahren nach einem der Ansprüche 1 bis 5, wobei die Weinbaubiomasse aus Traubentrub oder Traubentrester aus dem Elsass oder aus Burgund und/oder Spülwässern von Weinpressen ausgewählt wird.

7. Verfahren nach Anspruch 6, wobei die Elsass-Rebsorte von den Rebsorten Muscat, Auxerrois, Pinot gris, Gewürztraminer, Riesling stammt und die Burgund-Rebsorte von den Rebsorten Pinot noir, Chardonnay, Pinot noir/Chardonnay oder Aligoté stammt.

8. Verfahren nach einem der Ansprüche 1 bis 7, wobei die Fermentation bei 37°C ± 2°C durchgeführt wird.

9. Verfahren nach einem der Ansprüche 1 bis 8, wobei die Weinbiomasse vor der Verwendung durch Kühlung oder Gefrieren konserviert wird.

10. Verfahren nach einem der Ansprüche 1 bis 9, wobei der pH-Wert der Biomasse während der Fermentation mehr als 5,5 ± 0,2 beträgt.

11. Verfahren nach einem der Ansprüche 1 bis 10, wobei das durch die Fermentation der Biomasse produzierte Gas durch ein Spülgas extrahiert wird.

12. Verfahren nach einem der Ansprüche 1 bis 11, das ferner einen Schritt der Isolation der in der Biomasse nach oder während der Fermentation enthaltenen Mikrobenpopulation umfasst.

13. Verfahren zum Erhalten der Mikrobenpopulation, die in der gemäß dem Verfahren nach Anspruch 12 fermentierten Biomasse vorliegt.

## Claims

1. Method for producing hydrogen comprising the dark fermentation of wine biomass, without adding a microbial consortium and without thermal pretreatment, and in which the gas produced by said fermentation of the biomass is extracted.

2. Method according to claim 1, in which no additional nutritional intake is provided to the biomass.

3. Method according to either of claim 1 or 2, in which the wine biomass is chosen from grape sludge, grape marc, filter cakes, wine lees, spent marc, rinsing water from harvesting, sorting and winemaking equipment.

4. Method according to any of claims 1 to 3, in which the biomass used is composed of one or more sources of different wine biomass.

5. Method according to any of claims 1 to 4, in which the biomass comes from the wine-producing industry and concerns the grapes used for wine-making by pressing the harvest, regardless the soil, the colour of the grapes and the grape variety.

6. Method according to any of claims 1 to 5, in which the wine biomass is chosen from Alsace or Burgundy grape sludge or marc, and/or press rinsing water.

7. Method according to claim 6, in which the Alsace grape comes from Muscat, Auxerrois, Pinot gris, Gewurztraminer, Riesling grape varieties and the Burgundy grape comes from Pinot noir, Chardonnay, Pinot noir/Chardonnay or Aligoté grape varieties.

8. Method according to any of claims 1 to 7, in which the fermentation is carried out at 37°C ± 2°C.

9. Method according to any of claims 1 to 8, in which the wine biomass is preserved by refrigeration or freezing before use.

10. Method according to any of claims 1 to 9, in which the pH of the biomass is greater than 5.5 ± 0.2 during fermentation.

11. Method according to any of claims 1 to 10, in which the gas produced by the fermentation of biomass is extracted by a sweeping gas.

12. Method according to any of claims 1 to 11, further comprising a step of isolating the microbial consortium contained in the fermented or fermenting biomass.

13. Method for obtaining the microbial consortium present in the fermented biomass according to the method of claim 12.
